# EUROPEAN PATENT APPLICATION

(11) **EP 4 825 838 A1**
(43) Date of publication of application: **21.10.2026**
(21) Application number: 24902956.2
(22) Date of filing: 13.12.2024
(51) Int. Cl.: C07C 229/16, A61K 47/00

(54) **CATIONIC LIPID AND USE THEREOF**

(30) Priority: 13.12.2023 CN 202311715783; 11.06.2024 CN 202410749901; 23.08.2024 CN 202411170971
(71) Applicant: Shanghai Regenelead Therapies Co., Ltd., Shanghai 201206 (CN)
(72) Inventor: LIU, Chongyi, Shanghai 201206 (CN); JIANG, Jun, Shanghai 201206 (CN); HUANG, Jian, Shanghai 201206 (CN); ZHU, Lingjian, Shanghai 201206 (CN); LI, Tingting, Shanghai 201206 (CN); ZOU, Yang, Shanghai 201206 (CN); NING, Wei, Shanghai 201206 (CN); LIAO, Cheng, Shanghai 201206 (CN)
(74) Representative: Dragotti & Associati S.P.A.
(86) International application number: PCT/CN2024/139148
(87) International publication number: WO 2025/124541

(57) **Abstract**

The present disclosure relates to a cationic lipid and use thereof. Specifically provided is a compound represented by formula I or a salt thereof, wherein R¹ to R⁴, H¹, H², and L¹ are as defined in the present disclosure. The cationic lipid is used for delivering polynucleotides or nucleic acids and protecting the polynucleotides or nucleic acids from being degraded and removed in serum, and has excellent delivery efficiency.

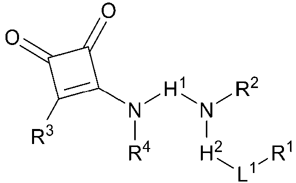

## Description

### TECHNICAL FIELD

The disclosure pertains to the field of pharmaceuticals and relates to a cationic lipid and use thereof.

### BACKGROUND

Gene therapy involves introducing exogenous normal genes into target cells to correct or compensate for diseases caused by defective and abnormal genes, achieving therapeutic purposes. Since the COVID-19 pandemic, gene therapy has become well-known to the public and has emerged as another hot topic following immunotherapy for cancer. It is gradually becoming a reality.

However, naked therapeutic genes are easily degraded by nucleases, exhibiting poor cellular uptake efficiency. Therefore, the preparation of a safe and efficient gene vector becomes a necessary condition for successful gene therapy. Mainstream nucleic acid drug delivery systems are divided into viral vector systems and non-viral systems. Lipid nanoparticle-mediated nucleic acid drug delivery is a primary method in non-viral delivery systems.

Nanoparticles consisting of cationic lipids and other helper lipids, such as lipid nanoparticles formed from cholesterol, phospholipids, and PEGylated lipids, can encapsulate nucleic acids to protect them from being degraded while promoting cellular uptake and reducing immune responses. In addition, lipid nanoparticles offer other advantages for the delivery of bioactive ingredients into cells, including good targeting, minimal side effects, good stability, efficient transfection, etc.

The rapidly advancing field of nucleic acid molecule-based treatment has placed higher demands on the delivery of nucleic acid drugs. Consequently, there remains a need to develop new cationic lipids and lipid nanoparticles for nucleic acid delivery. Cationic lipids that provide excellent drug-to-lipid ratios, protect nucleic acids from degradation and clearance in serum, are suitable for systemic or local delivery, and enable intracellular delivery of nucleic acids are preferred.

### SUMMARY

The disclosure provides a compound represented by formula I or a salt thereof, 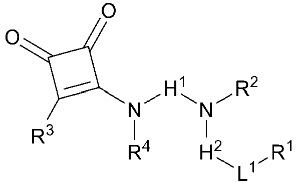
wherein H¹ is selected from the group consisting of C₁₋₁₂ alkylene, C₂₋₁₂ alkenylene, C₃₋₈ cycloalkylene, and C₃₋₈ cycloalkenylene;
H² is selected from the group consisting of C₁₋₁₂ alkylene and C₁₋₁₂ heteroalkylene;
provided that when H² is C₁₋₁₂ alkylene, H¹ is selected from the group consisting of C₃₋₈ cycloalkylene and C₃₋₈ cycloalkenylene;
R¹ is selected from the group consisting of C₁₋₂₄ alkyl and C₂₋₂₄ alkenyl;
R² is selected from the group consisting of C₁₋₂₄ alkyl, C₁₋₂₄ alkenyl, and -R^{b}-L²-R^{c};
each L¹ or L² is independently selected from the group consisting of -C(O)O-, -OC(O)-, -C(O)-, -OC(O)O-, -O-, -S(O)q-, -S-S-, -C(O)S-, -SC(O)-, -NR^{a}C(O)-, -C(O)NR^{a}-, - NR^{a}C(O)NR^{a}-, -NR^{a}C(O)O-, -OC(O)NR^{a}-, -P(O)(OR^{a}), and a bond, wherein R^{a} is selected from the group consisting of hydrogen and C₁₋₆ alkyl, and q is selected from the group consisting of 0, 1, and 2;
R^{b} is selected from the group consisting of C₁₋₁₂ alkylene and C₂₋₁₂ alkenylene;
R^{c} is selected from the group consisting of C₁₋₂₄ alkyl and C₂₋₂₄ alkenyl;
R³ is selected from the group consisting of alkylamino and cycloalkylamino;
R⁴ is selected from the group consisting of hydrogen and C₁₋₆ alkyl.

In some embodiments, in the compound represented by formula I or the salt thereof, H² is heteroalkylene, wherein the heteroalkylene comprises at least one heteroatom selected from the group consisting of O, N, and S.

In some embodiments, in the compound represented by formula I or the salt thereof, H² is C₁₋₁₂ heteroalkylene, such as C₂₋₉ heteroalkylene, C₂₋₇ heteroalkylene, C₂₋₄ heteroalkylene, C₃₋₅ heteroalkylene, or C₃₋₆ heteroalkylene.

In some embodiments, in the compound represented by formula I or the salt thereof, the heteroalkylene is heteroalkylene comprising at least one oxygen atom. In some embodiments, in the compound represented by formula I or the salt thereof, the heteroalkylene is heteroalkylene comprising two oxygen atoms. In some embodiments, in the compound represented by formula I or the salt thereof, the heteroalkylene is heteroalkylene comprising at least one nitrogen atom. In some embodiments, in the compound represented by formula I or the salt thereof, the heteroalkylene is heteroalkylene comprising at least one oxygen atom.

In some embodiments, in the compound represented by formula I or the salt thereof, R³ is alkylamino, such as methylamino (CH₃NH-) or dimethylamino ((CH₃)₂N-).

In some embodiments, in the compound represented by formula I or the salt thereof, R¹ is C₁₋₂₄ alkyl, including C₂ alkyl, C₃ alkyl, C₄ alkyl, Cs alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, or C₂₃ alkyl.

In some other embodiments, in the compound represented by formula I or the salt thereof, R¹ is C₄-₁₈ alkyl.

In some embodiments, in the compound represented by formula I or the salt thereof, R¹ is linear C₄-₁₈ alkyl.

In some embodiments, in the compound represented by formula I or the salt thereof, R¹ is branched C₄-₁₈ alkyl.

In some embodiments, in the compound represented by formula I or the salt thereof, R¹ is branched C₁₇ alkyl.

In some other embodiments, in the compound represented by formula I or the salt thereof, R¹ is C₂₋₂₄ alkenyl, including C₂ alkenyl, C₃ alkenyl, C₄ alkenyl, Cs alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, or C₂₁ alkenyl. In the compound represented by formula I or the salt thereof provided in some other embodiments, R¹ is C₄-₁₈ alkenyl.

In some embodiments, in the compound represented by formula I or the salt thereof, R¹ is linear C₄-₁₈ alkenyl.

In some embodiments, in the compound represented by formula I or the salt thereof, R¹ is branched C₄-₁₈ alkenyl.

In the compound represented by formula I or the salt thereof provided in some other embodiments, R² is -R^{b}-L²-R^{c}, wherein R^{b}, L², or R^{c} is as defined above.

Further, the compound represented by formula I provided in some embodiments is a compound represented by formula IIa: 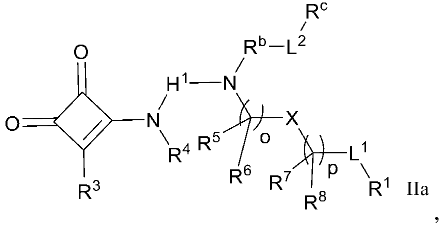 wherein X is -N(R^{d})- or -O-; o is selected from the group consisting of integers between 1 and 11, p is selected from the group consisting of integers between 1 and 5, and o + p is not greater than 12; R^{d} is selected from the group consisting of hydrogen and C₁₋₆ alkyl; R⁵, R⁶, R⁷, and R⁸ are each independently selected from the group consisting of hydrogen, halogen, hydroxy, and C₁₋₆ alkyl; L¹, L², H¹, R¹, R³, R⁴, R^{b}, and R^{c} are as defined in the compound represented by formula I.

In some embodiments, in the compound represented by formula I or IIa or the salt thereof, R^{b} is C₁₋₁₂ alkylene, including C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula I or IIa or the salt thereof, R^{b} is linear C₃₋₁₂ alkylene.

In some embodiments, in the compound represented by formula I or IIa or the salt thereof, R^{b} is branched C₃₋₁₂ alkylene.

In some embodiments, in the compound represented by formula I or IIa or the salt thereof, R^{b} is linear C₇ alkylene.

In some embodiments, in the compound represented by formula I or IIa or the salt thereof, R^{b} is linear C₈ alkylene.

In some embodiments, in the compound represented by formula I or IIa or the salt thereof, R^{b} is linear C₉ alkylene.

In another aspect, in the compound represented by formula I or IIa or the salt thereof, R^{b} is C₁₋₁₂ alkenylene, including C₃ alkenylene, C₄ alkenylene, C₅ alkenylene, C₆ alkenylene, C₇ alkenylene, C₈ alkenylene, C₉ alkenylene, C₁₀ alkenylene, C₁₁ alkenylene, or C₁₂ alkenylene.

In some embodiments, in the compound represented by formula I or IIa or the salt thereof, R^{b} is branched C₃₋₁₂ alkenylene.

In some embodiments, in the compound represented by formula I or IIa or the salt thereof, R^{b} is linear C₇ alkenylene.

In some embodiments, in the compound represented by formula I or IIa or the salt thereof, R^{b} is linear C₈ alkenylene.

In some embodiments, in the compound represented by formula I or IIa or the salt thereof, R^{b} is linear C₉ alkenylene.

In some other embodiments, in the compound represented by formula I or IIa or the salt thereof, R^{c} is C₁₋₂₄ alkyl, including C₂ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, C₂₃ alkyl, or C₂₄ alkyl.

In some embodiments, in the compound represented by formula I or IIa or the salt thereof, R^{c} is linear C₁₋₁₂ alkyl.

In some embodiments, in the compound represented by formula I or IIa or the salt thereof, R^{c} is branched C₁₋₁₂ alkyl.

In some embodiments, in the compound represented by formula I or IIa or the salt thereof, R^{c} is branched C₁₁ alkyl.

In some embodiments, in the compound represented by formula I or IIa or the salt thereof, R^{c} is branched C₁₇ alkyl.

In some embodiments, in the compound represented by formula I or IIa or the salt thereof, R^{c} is linear C₉ alkyl.

In some other embodiments, in the compound represented by formula I or IIa or the salt thereof, R^{c} is C₂₋₂₄ alkenyl, including C₂ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₁ alkenyl, C₂₂ alkenyl, C₂₃ alkenyl, or C₂₄ alkenyl.

In some embodiments, in the compound represented by formula I or IIa or the salt thereof, R^{c} is linear C₁₋₁₂ alkenyl.

In some embodiments, in the compound represented by formula I or IIa or the salt thereof, R^{c} is branched C₁₋₁₂ alkenyl.

In some embodiments, in the compound represented by formula I or IIa or the salt thereof, R^{c} is branched C₁₁ alkenyl.

In some embodiments, in the compound represented by formula I or IIa or the salt thereof, R^{c} is branched C₁₇ alkenyl.

In some embodiments, in the compound represented by formula I or IIa or the salt thereof, R^{c} is linear C₉ alkenyl.

In some embodiments, in the compound represented by formula I or IIa or the salt thereof, p is selected from the group consisting of 1 and 2.

In some embodiments, in the compound represented by formula I or IIa or the salt thereof, o is selected from the group consisting of integers between 4 and 7, such as 5 or 6.

In some embodiments, in the compound represented by formula I or IIa or the salt thereof, X is -O-.

In some embodiments, in the compound represented by formula I or IIa or the salt thereof, p is selected from the group consisting of 1 and 2, and X is -O-.

In some embodiments, in the compound represented by formula I or II or the salt thereof, p is selected from the group consisting of 3 and 4, o is selected from the group consisting of 5 and 6, and X is -O-.

In some embodiments, in the compound represented by formula I or IIa or the salt thereof, R⁵ and R⁶ are each independently C₁₋₆ alkyl, such as methyl, ethyl, or propyl.

In some embodiments, in the compound represented by formula I or IIa or the salt thereof, R⁷ and R⁸ are each independently C₁₋₆ alkyl, such as methyl, ethyl, or propyl.

In some embodiments, in the compound represented by formula I or IIa or the salt thereof, R⁵, R⁶, R⁷, and R⁸ are each independently hydrogen.

In some embodiments, in the compound represented by formula I or II or the salt thereof, p is selected from the group consisting of 3 and 4, and o is selected from the group consisting of 5 and 6; X is -O-; R⁵, R⁶, R⁷, and R⁸ are each independently hydrogen.

In some embodiments, in the compound represented by formula I or IIa or the salt thereof, L¹ and L² are each independently selected from the group consisting of -C(O)O-, -OC(O)-, and a bond.

In some embodiments, in the compound represented by formula I or IIa or the salt thereof, R⁴ is C₁₋₆ alkyl, such as methyl or ethyl.

In some embodiments, in the compound represented by formula I or IIa or the salt thereof, R⁴ is hydrogen.

In some embodiments, in the compound represented by formula I or II or the salt thereof, p is selected from the group consisting of 3 and 4, and o is selected from the group consisting of 5 and 6; X is -O-; R⁵, R⁶, R⁷, and R⁸ are each independently hydrogen, and R⁴ is hydrogen.

In some embodiments, in the compound represented by formula I or II or the salt thereof, p is selected from the group consisting of 1 and 2, and o is selected from the group consisting of 5 and 6; X is -O-; R⁵, R⁶, R⁷, and R⁸ are each independently hydrogen, and R⁴ is hydrogen. In some embodiments, in the compound represented by formula I or II or the salt thereof, p is selected from the group consisting of 1 and 2, and o is selected from the group consisting of 5 and 6; X is -O-; R⁵, R⁶, R⁷, and R⁸ are each independently hydrogen, and R⁴ is hydrogen; L¹ and L² are each independently selected from the group consisting of -C(O)O- and -OC(O)-.

In some embodiments, in the compound represented by formula I or II or the salt thereof, o is selected from the group consisting of 1 and 2, and p is selected from the group consisting of 4 and 5; X is -O-; R⁵, R⁶, R⁷, and R⁸ are each independently hydrogen, and R⁴ is hydrogen.

In some embodiments, in the compound represented by formula I or II or the salt thereof, o is selected from the group consisting of 1 and 2, and p is selected from the group consisting of 4 and 5; X is -O-; R⁵, R⁶, R⁷, and R⁸ are each independently hydrogen, and R⁴ is hydrogen; L¹ and L² are each independently selected from the group consisting of - C(O)O- and -OC(O)-.

In some embodiments, the compound represented by formula IIa is a compound represented by formula IIa-1 or a compound represented by formula IIa-2:

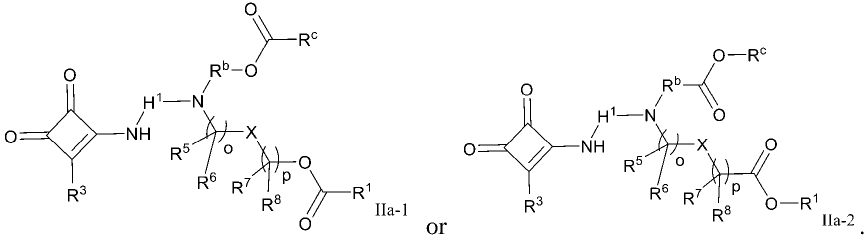

In some of the embodiments, in the compound represented by formula IIa-1 or IIa-1 or the salt thereof, o is selected from the group consisting of 1 and 2, and p is selected from the group consisting of 4 and 5; X is -O-; R⁵, R⁶, R⁷, and R⁸ are each independently hydrogen.

In some of the embodiments, in the compound represented by formula IIa-1 or IIa-1 or the salt thereof, p is selected from the group consisting of 1 and 2, and o is selected from the group consisting of 4 and 5; X is -O-; R⁵, R⁶, R⁷, and R⁸ are each independently hydrogen.

In another aspect, in certain embodiments, in the compound represented by formula I or IIa or the salt thereof, L¹ and L² are each independently selected from the group consisting of -C(O)-, -OC(O)O-, -O-, and a bond.

In certain embodiments, in the compound represented by formula I or IIa or the salt thereof, L¹ and L² are each independently selected from the group consisting of -S(O)_{q}-, -S-S-, -C(O)S-, -SC(O)-, and a bond.

In certain embodiments, in the compound represented by formula I or IIa or the salt thereof, L¹ and L² are each independently selected from the group consisting of - NR^{a}C(O)-, -C(O)NR^{a}-, -NR^{a}C(O)NR^{a}-, -NR^{a}C(O)O-, and a bond.

In certain embodiments, in the compound represented by formula I or IIa or the salt thereof, L¹ and L² are each independently selected from the group consisting of - NR^{a}C(O)NR^{a}-, -NR^{a}C(O)O-, -OC(O)NR^{a}-, and a bond.

In some embodiments, in the compound represented by formula I or IIa, H¹ is selected from the group consisting of C₁₋₁₂ alkylene and C₂₋₁₂ alkenylene.

In some embodiments, in the compound represented by formula I or IIa, H¹ is C₁₋₁₂ alkylene, including C₂ alkylene, C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula I or IIa, H¹ is C₃ alkylene, such as linear C₃ alkylene.

In some embodiments, in the compound represented by formula I or IIa, H¹ is C₄ alkylene, such as linear C₃ alkylene.

In some embodiments, in the compound represented by formula I or IIa, H¹ is C₅ alkylene, such as linear C₃ alkylene.

In some embodiments, in the compound represented by formula I or II or the salt thereof, p is selected from the group consisting of 3 and 4, and o is selected from the group consisting of 5 and 6; X is -O-; R⁵, R⁶, R⁷, and R⁸ are each independently hydrogen; H¹ is linear C₃ alkylene; R^{b} is linear C₆₋₈ alkylene.

In another aspect, in the compound represented by formula I, H¹ is C₃₋₈ cycloalkylene, such as cyclohexylene.

In some embodiments, the compound represented by formula IIa is a compound represented by formula IIa-1a or a compound represented by formula IIa-2a:

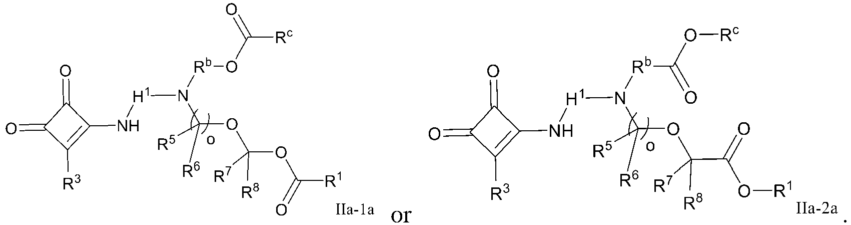

In some embodiments, in the compound represented by formula IIa-1 or IIa-2 or IIa-1a or IIa-2a or the salt thereof, R⁷ and R⁸ are each independently hydrogen.

In some embodiments, in the compound represented by formula IIa-1 or IIa-2 or IIa-1a or IIa-2a or the salt thereof, o is selected from the group consisting of 5 and 6.

In some embodiments, in the compound represented by formula IIa-1 or IIa-2 or IIa-1a or IIa-2a or the salt thereof, R⁵ and R⁶ are each independently hydrogen.

In some embodiments, in the compound represented by formula IIa-1 or IIa-2 or IIa-1a or IIa-2a or the salt thereof, H¹ is C₃ alkylene, such as linear C₃ alkylene.

In some embodiments, in the compound represented by formula IIa-1 or IIa-2 or IIa-1a or IIa-2a or the salt thereof, H¹ is C₃ alkylene, such as linear C₃ alkylene.

In some embodiments, in the compound represented by formula IIa-1 or IIa-2 or IIa-1a or IIa-2a or the salt thereof, R^{b} is branched C₃₋₁₂ alkylene.

In some embodiments, in the compound represented by formula IIa-1 or IIa-2 or IIa-1a or IIa-2a or the salt thereof, R^{b} is linear C₇ alkylene.

In some embodiments, in the compound represented by formula IIa-1 or IIa-2 or IIa-1a or IIa-2a or the salt thereof, R^{c} is

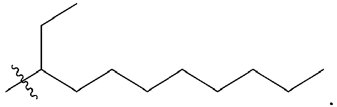

In some embodiments, the compound represented by formula IIa or IIa-2 or the salt thereof is a compound represented by formula IIa-3 or a salt thereof: 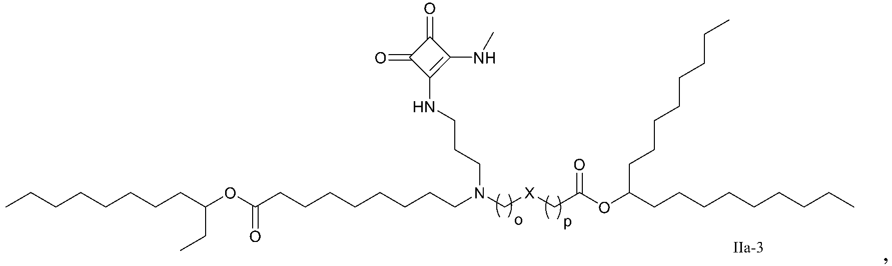 wherein o and p are each independently selected from the group consisting of integers between 1 and 6, and o + p = 6 or 7; X is -N(R^{d})- or -O-, wherein R^{d} is selected from the group consisting of hydrogen and C₁₋₆ alkyl.

In some embodiments, in the compound represented by formula IIa-3 or the salt thereof, o is selected from the group consisting of 1 and 2, p is selected from the group consisting of 4 and 5, and X is -O-.

In some embodiments, in the compound represented by formula IIa-3 or the salt thereof, p is selected from the group consisting of 1 and 2, o is selected from the group consisting of 4 and 5, and X is -O-.

In some embodiments, the compound represented by formula I or the salt thereof is not 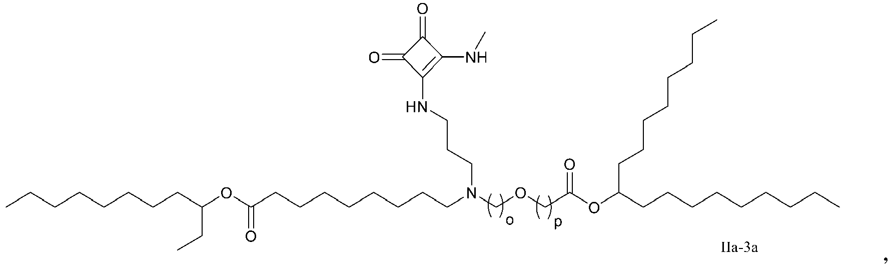 wherein o = 3, and p = 3.

In some embodiments, the compound represented by formula I is a compound represented by formula IIb: 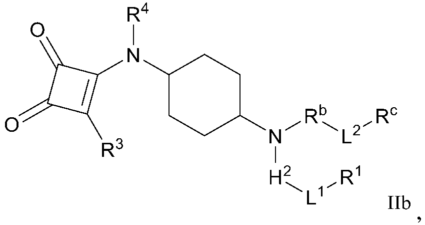 wherein L¹, L², H², R¹, R³, R⁴, R^{b}, and R^{c} are as defined in the compound represented by formula I.

In some other embodiments, in the compound represented by formula I or IIb or the salt thereof, L¹ and L² are each independently selected from the group consisting of -OC(O)O-and a bond.

In some other embodiments, in the compound represented by formula I or IIb or the salt thereof, R⁴ is hydrogen.

In some embodiments, the compound represented by formula IIb is a compound represented by formula IIb-1 or a compound represented by formula IIb-2:

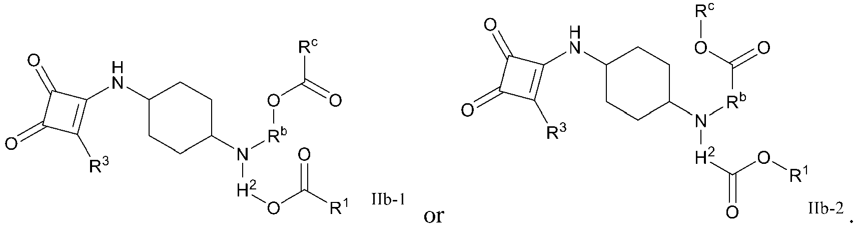

In certain embodiments, in the compound represented by formula I or IIb or the salt thereof, L¹ and L² are each independently selected from the group consisting of -C(O)-, - OC(O)O-, -O-, and a bond.

In certain embodiments, in the compound represented by formula I or IIb or the salt thereof, L¹ and L² are each independently selected from the group consisting of -S(O)_{q}-, -S-S-, -C(O)S-, -SC(O)-, and a bond.

In certain embodiments, in the compound represented by formula I or IIb or the salt thereof, L¹ and L² are each independently selected from the group consisting of - NR^{a}C(O)-, -C(O)NR^{a}-, -NR^{a}C(O)NR^{a}-, -NR^{a}C(O)O-, and a bond.

In certain embodiments, in the compound represented by formula I or IIb or the salt thereof, L¹ and L² are each independently selected from the group consisting of - NR^{a}C(O)NR^{a}-, -NR^{a}C(O)O-, -OC(O)NR^{a}-, and a bond.

In some other embodiments, in the compound represented by formula I or IIb or the salt thereof, H² is C₁₋₁₂ alkylene, including C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula I or IIb or the salt thereof, H² is linear C₃₋₁₂ alkylene.

In some embodiments, in the compound represented by formula I or IIb or the salt thereof, H² is branched C₃₋₁₂ alkylene.

In some embodiments, in the compound represented by formula I or IIb or the salt thereof, H² is linear C₇ alkylene.

In some embodiments, in the compound represented by formula I or IIb or the salt thereof, H² is linear C₈ alkylene.

In some embodiments, in the compound represented by formula I or IIb or the salt thereof, H² is linear C₉ alkylene.

In another aspect, in some embodiments, in the compound represented by formula IIb or the salt thereof, H² is C₁₋₁₂ heteroalkylene, such as C₂₋₉ heteroalkylene, C₂₋₇ heteroalkylene, C₂₋₄ heteroalkylene, C₃₋₅ heteroalkylene, or C₃₋₆ heteroalkylene.

In some embodiments, in the compound represented by formula IIb or the salt thereof, the heteroalkylene is heteroalkylene comprising at least one oxygen atom. In some embodiments, in the compound represented by formula IIb or the salt thereof, the heteroalkylene is heteroalkylene comprising two oxygen atoms. In some embodiments, in the compound represented by formula IIb or the salt thereof, the heteroalkylene is heteroalkylene comprising at least one nitrogen atom. In some embodiments, in the compound represented by formula IIb or the salt thereof, the heteroalkylene is heteroalkylene comprising at least one oxygen atom.

In certain embodiments, in the compound represented by formula I or IIb or the salt thereof, R^{b} is linear C₃₋₁₂ alkylene, and H² is C₁₋₁₂ heteroalkylene, such as C₇ alkylene or C₈ alkylene.

In certain embodiments, in the compound represented by formula I or IIb or the salt thereof, R^{b} is linear C₃₋₁₂ alkylene, and H² is C₁₋₁₂ alkylene, such as C₇ alkylene or C₈ alkylene.

In the compound represented by formula I provided in some embodiments, R^{c} is selected from the group consisting of: 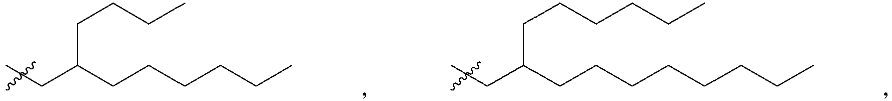 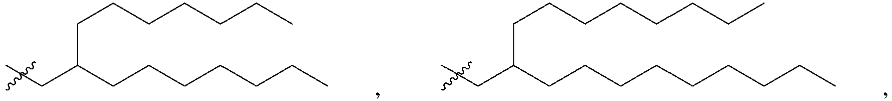 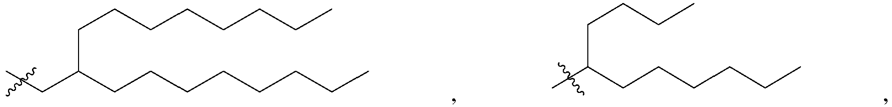 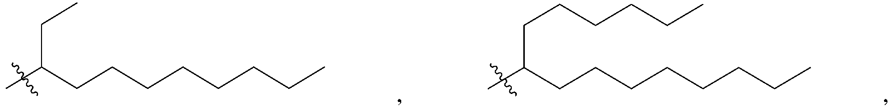 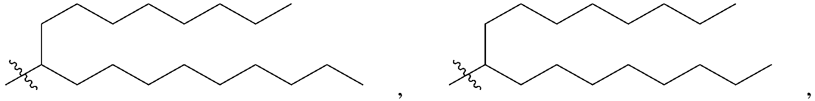 and

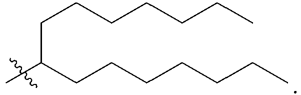

In another aspect, in the compound represented by formula I provided in some embodiments, R¹ is selected from the group consisting of: 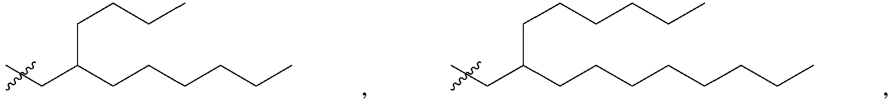 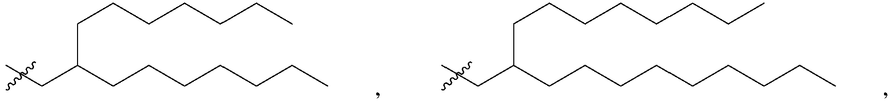 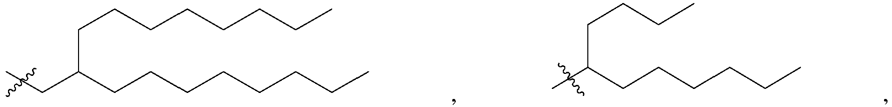 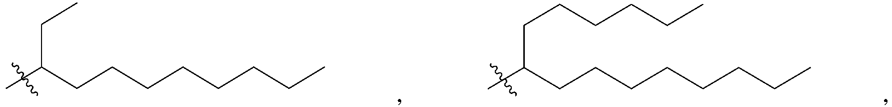 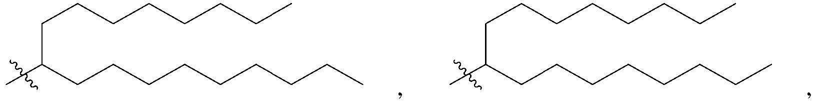 and

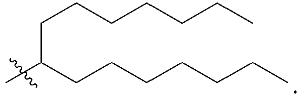

Typical compounds represented by formula I include:

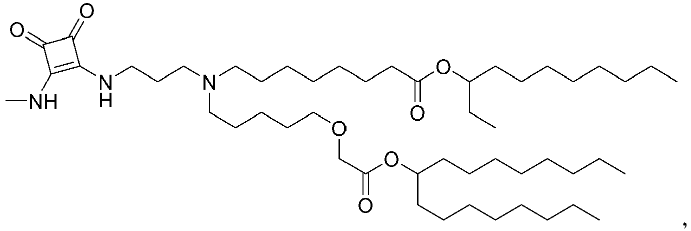

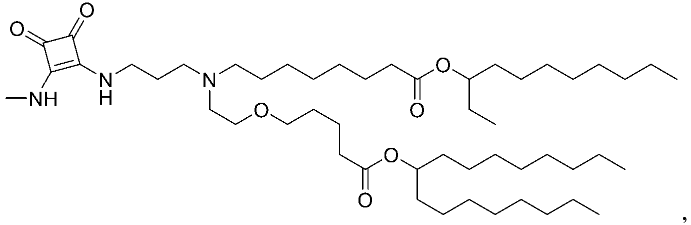

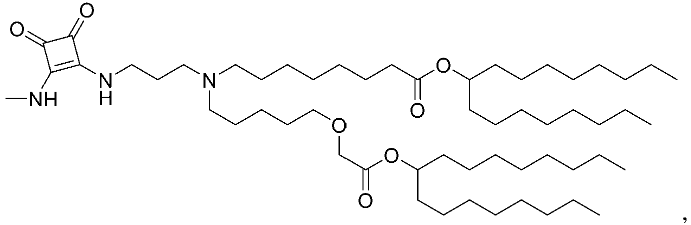

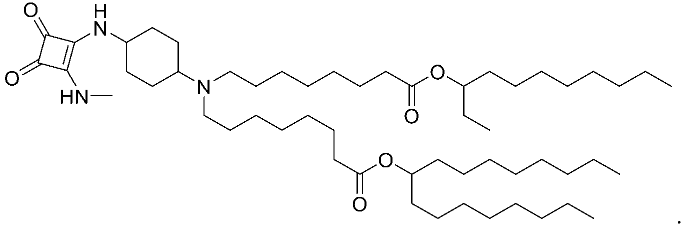

In another aspect, the disclosure further provides a compound represented by formula A or a salt thereof: 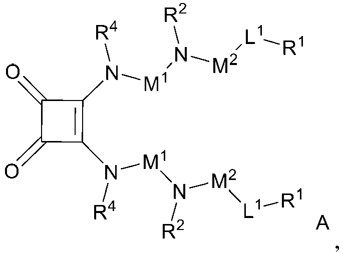 wherein each M¹ is independently selected from the group consisting of C₁₋₁₂ alkylene, C₂₋₁₂ alkenylene, C₃₋₈ cycloalkylene, and C₃₋₈ cycloalkenylene;
each M² is independently selected from the group consisting of C₁₋₁₂ alkylene and C₁₋₁₂ heteroalkylene;
each R¹ is independently selected from the group consisting of C₁₋₂₄ alkyl and C₂₋₂₄ alkenyl;
each R² is independently selected from the group consisting of C₁₋₂₄ alkyl, C₁₋₂₄ alkenyl, and -R^{b}-L²-R^{c};
each L¹ or L² is independently selected from the group consisting of -C(O)O-, -OC(O)-, -C(O)-, -OC(O)O-, -O-, -S(O)q-, -S-S-, -C(O)S-, -SC(O)-, -NR^{a}C(O)-, -C(O)NR^{a}-, - NR^{a}C(O)NR^{a}-, -NR^{a}C(O)O-, -OC(O)NR^{a}-, -P(O)(OR^{a}), and a bond, wherein R^{a} is selected from the group consisting of hydrogen and C₁₋₆ alkyl and C₂₋₆ alkenyl, and q is selected from the group consisting of 0, 1, and 2;
each R^{b} is independently selected from the group consisting of C₁₋₁₂ alkylene and C₂₋₁₂ alkenylene;
each R^{c} is independently selected from the group consisting of C₁₋₂₄ alkyl and C₂₋₂₄ alkenyl;
each R⁴ is independently selected from the group consisting of hydrogen and C₁₋₆ alkyl.

In some embodiments, in the compound represented by formula A or the salt thereof, each M² is independently C₁₋₁₂ alkylene, including C₂ alkylene, C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula A or the salt thereof, each M² is independently C₇ alkylene, such as linear C₇ alkylene.

In some embodiments, in the compound represented by formula A or the salt thereof, each M² is independently C₈ alkylene, such as linear C₈ alkylene.

In some other embodiments, in the compound represented by formula A or the salt thereof, each M² is independently heteroalkylene, wherein the heteroalkylene comprises at least one heteroatom selected from the group consisting of O, N, and S.

In certain embodiments, in the compound represented by formula A or the salt thereof, each M² is independently C₁₋₁₂ heteroalkylene, such as C₂₋₉ heteroalkylene, C₂₋₇ heteroalkylene, C₂₋₄ heteroalkylene, C₃₋₅ heteroalkylene, or C₃₋₆ heteroalkylene.

In another aspect, in some embodiments, in the compound represented by formula A, each M¹ is independently selected from the group consisting of C₁₋₁₂ alkylene and C₂₋₁₂ alkenylene.

In some embodiments, in the compound represented by formula A, each M¹ is independently C₁₋₁₂ alkylene, including C₂ alkylene, C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula A, each M¹ is independently C₃ alkylene, such as linear C₃ alkylene.

In some embodiments, in the compound represented by formula A, each M¹ is independently C₄ alkylene, such as linear C₃ alkylene.

In some embodiments, in the compound represented by formula A, each M¹ is independently C₅ alkylene, such as linear C₃ alkylene.

In the compound represented by formula A or the salt thereof provided in some other embodiments, R² is -R^{b}-L²-R^{c}, wherein R^{b}, L², and R^{c} are as defined above.

In some embodiments, in the compound represented by formula A or the salt thereof, L¹ and L² are each independently selected from the group consisting of -C(O)O-, -OC(O)-, and a bond.

In some embodiments, the compound represented by formula A is a compound represented by formula Aa-1 or formula Aa-2:

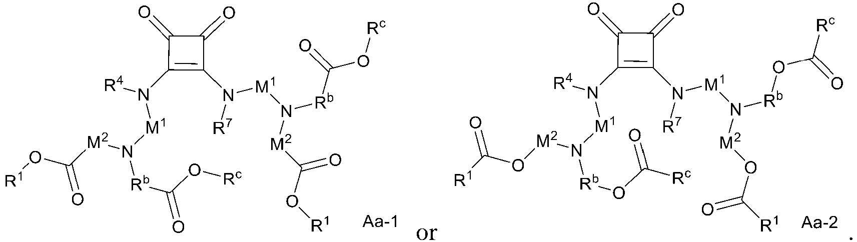

In certain embodiments, in the compound represented by formula A or the salt thereof, L¹ and L² are each independently selected from the group consisting of -C(O)-, -OC(O)O-, -O-, and a bond.

In certain embodiments, in the compound represented by formula A or the salt thereof, L¹ and L² are each independently selected from the group consisting of -S(O)_{q}-, -S-S-, - C(O)S-, -SC(O)-, and a bond.

In certain embodiments, in the compound represented by formula A or the salt thereof, L¹ and L² are each independently selected from the group consisting of -NR^{a}C(O)-, - C(O)NR^{a}-, -NR^{a}C(O)NR^{a}-, -NR^{a}C(O)O-, and a bond.

In certain embodiments, in the compound represented by formula A or the salt thereof, L¹ and L² are each independently selected from the group consisting of -NR^{a}C(O)NR^{a}-, - NR^{a}C(O)O-, -OC(O)NR^{a}-, and a bond.

In another aspect, in the compound represented by formula A, each M¹ is independently C₃₋₈ cycloalkylene, such as cyclohexylene.

In some embodiments, the compound represented by formula A is a compound represented by formula Ab-1 or formula Ab-2:

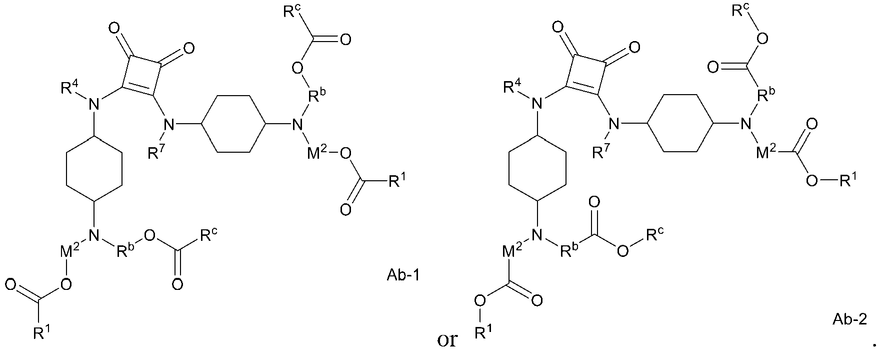

In some embodiments, in the compound represented by formula A or formula Aa-1 or formula Aa-2 or formula Ab-1 or formula Ab-2 or the salt thereof, each R¹ is independently C₁₋₂₄ alkyl, including C₂ alkyl, C₃ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, or C₂₃ alkyl.

In some other embodiments, in the compound represented by formula A or formula Aa-1 or formula Aa-2 or formula Ab-1 or formula Ab-2 or the salt thereof, each R¹ is independently C₄-₁₈ alkyl.

In some embodiments, in the compound represented by formula A or formula Aa-1 or formula Aa-2 or formula Ab-1 or formula Ab-2 or the salt thereof, R¹ is linear C₄-₁₈ alkyl. In some embodiments, in the compound represented by formula A or formula Aa-1 or formula Aa-2 or formula Ab-1 or formula Ab-2 or the salt thereof, R¹ is branched C₄-₁₈ alkyl.

In some embodiments, in the compound represented by formula A or formula Aa-1 or formula Aa-2 or formula Ab-1 or formula Ab-2 or the salt thereof, R¹ is branched C₁₇ alkyl.

In some other embodiments, in the compound represented by formula A or formula Aa-1 or formula Aa-2 or formula Ab-1 or formula Ab-2 or the salt thereof, each R¹ is independently C₂₋₂₄ alkenyl, including C₂ alkenyl, C₃ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, C₂₀ alkenyl, or C₂₁ alkenyl.

In the compound represented by formula I or the salt thereof provided in some other embodiments, R¹ is C₄-₁₈ alkenyl.

In some embodiments, in the compound represented by formula A or formula Aa-1 or formula Aa-2 or formula Ab-1 or formula Ab-2 or the salt thereof, R¹ is linear C₄-₁₈ alkenyl.

In some embodiments, in the compound represented by formula A or formula Aa-1 or formula Aa-2 or formula Ab-1 or formula Ab-2 or the salt thereof, R¹ is branched C₄-₁₈ alkenyl.

In some embodiments, in the compound represented by formula A or formula Aa-1 or formula Aa-2 or formula Ab-1 or formula Ab-2 or the salt thereof, each R^{b} is independently C₁₋₁₂ alkylene, including C₃ alkylene, C₄ alkylene, C₅ alkylene, C₆ alkylene, C₇ alkylene, C₈ alkylene, C₉ alkylene, C₁₀ alkylene, C₁₁ alkylene, or C₁₂ alkylene.

In some embodiments, in the compound represented by formula A or formula Aa-1 or formula Aa-2 or formula Ab-1 or formula Ab-2 or the salt thereof, R^{b} is linear C₃₋₁₂ alkylene.

In some embodiments, in the compound represented by formula A or formula Aa-1 or formula Aa-2 or formula Ab-1 or formula Ab-2 or the salt thereof, R^{b} is branched C₃₋₁₂ alkylene.

In some embodiments, in the compound represented by formula A or formula Aa-1 or formula Aa-2 or formula Ab-1 or formula Ab-2 or the salt thereof, R^{b} is linear C₇ alkylene. In some embodiments, in the compound represented by formula A or formula Aa-1 or formula Aa-2 or formula Ab-1 or formula Ab-2 or the salt thereof, R^{b} is linear C₈ alkylene. In some embodiments, in the compound represented by formula A or formula Aa-1 or formula Aa-2 or formula Ab-1 or formula Ab-2 or the salt thereof, R^{b} is linear C₉ alkylene. In another aspect, in the compound represented by formula A or formula Aa-1 or formula Aa-2 or formula Ab-1 or formula Ab-2 or the salt thereof, each R^{b} is independently C₂₋₁₂ alkenylene, including C₃ alkenylene, C₄ alkenylene, C₅ alkenylene, C₆ alkenylene, C₇ alkenylene, C₈ alkenylene, C₉ alkenylene, C₁₀ alkenylene, C₁₁ alkenylene, or C₁₂ alkenylene.

In some embodiments, in the compound represented by formula A or formula Aa-1 or formula Aa-2 or formula Ab-1 or formula Ab-2 or the salt thereof, R^{b} is branched C₃₋₁₂ alkenylene.

In some embodiments, in the compound represented by formula A or formula Aa-1 or formula Aa-2 or formula Ab-1 or formula Ab-2 or the salt thereof, R^{b} is linear C₇ alkenylene.

In some embodiments, in the compound represented by formula A or formula Aa-1 or formula Aa-2 or formula Ab-1 or formula Ab-2 or the salt thereof, R^{b} is linear C₈ alkenylene.

In some embodiments, in the compound represented by formula A or formula Aa-1 or formula Aa-2 or formula Ab-1 or formula Ab-2 or the salt thereof, R^{b} is linear C₉ alkenylene.

In some other embodiments, in the compound represented by formula A or formula Aa-1 or formula Aa-2 or formula Ab-1 or formula Ab-2 or the salt thereof, each R^{c} is independently C₁₋₂₄ alkyl, including C₂ alkyl, C₄ alkyl, C₅ alkyl, C₆ alkyl, C₇ alkyl, C₈ alkyl, C₉ alkyl, C₁₀ alkyl, C₁₁ alkyl, C₁₂ alkyl, C₁₃ alkyl, C₁₄ alkyl, C₁₅ alkyl, C₁₆ alkyl, C₁₇ alkyl, C₁₈ alkyl, C₁₉ alkyl, C₂₀ alkyl, C₂₁ alkyl, C₂₂ alkyl, C₂₃ alkyl, or C₂₄ alkyl.

In some embodiments, in the compound represented by formula A or formula Aa-1 or formula Aa-2 or formula Ab-1 or formula Ab-2 or the salt thereof, R^{c} is linear C₁₋₁₂ alkyl. In some embodiments, in the compound represented by formula A or formula Aa-1 or formula Aa-2 or formula Ab-1 or formula Ab-2 or the salt thereof, R^{c} is branched C₁₋₁₂ alkyl.

In some embodiments, in the compound represented by formula A or formula Aa-1 or formula Aa-2 or formula Ab-1 or formula Ab-2 or the salt thereof, R^{c} is branched C₁₁ alkyl.

In some embodiments, in the compound represented by formula A or formula Aa-1 or formula Aa-2 or formula Ab-1 or formula Ab-2 or the salt thereof, R^{c} is branched C₁₇ alkyl.

In some embodiments, in the compound represented by formula A or formula Aa-1 or formula Aa-2 or formula Ab-1 or formula Ab-2 or the salt thereof, R^{c} is linear C₉ alkyl.

In some other embodiments, in the compound represented by formula A or formula Aa-1 or formula Aa-2 or formula Ab-1 or formula Ab-2 or the salt thereof, each R^{c} is independently C₂₋₂₀ alkenyl, including C₂ alkenyl, C₄ alkenyl, C₅ alkenyl, C₆ alkenyl, C₇ alkenyl, C₈ alkenyl, C₉ alkenyl, C₁₀ alkenyl, C₁₁ alkenyl, C₁₂ alkenyl, C₁₃ alkenyl, C₁₄ alkenyl, C₁₅ alkenyl, C₁₆ alkenyl, C₁₇ alkenyl, C₁₈ alkenyl, C₁₉ alkenyl, or C₂₀ alkenyl. In some embodiments, in the compound represented by formula A or formula Aa-1 or formula Aa-2 or formula Ab-1 or formula Ab-2 or the salt thereof, R^{c} is linear C₁₋₁₂ alkenyl.

In some embodiments, in the compound represented by formula A or formula Aa-1 or formula Aa-2 or formula Ab-1 or formula Ab-2 or the salt thereof, R^{c} is branched C₁₋₁₂ alkenyl.

In some embodiments, in the compound represented by formula A or formula Aa-1 or formula Aa-2 or formula Ab-1 or formula Ab-2 or the salt thereof, R^{c} is branched C₁₁ alkenyl.

In some embodiments, in the compound represented by formula A or formula Aa-1 or formula Aa-2 or formula Ab-1 or formula Ab-2 or the salt thereof, R^{c} is branched C₁₇ alkenyl.

In some embodiments, in the compound represented by formula A or formula Aa-1 or formula Aa-2 or formula Ab-1 or formula Ab-2 or the salt thereof, R^{c} is linear C₉ alkenyl. In some embodiments, in the compound represented by formula A or formula Aa-1 or formula Aa-2 or formula Ab-1 or formula Ab-2 or the salt thereof, each R⁴ is independently hydrogen.

In some embodiments, in the compound represented by formula A or formula Aa-1 or formula Aa-2 or formula Ab-1 or formula Ab-2 or the salt thereof, each R⁴ is independently C₁₋₆ alkyl, such as methyl or ethyl.

In another aspect, in the compound represented by formula A or formula Aa-1 or formula Aa-2 or formula Ab-1 or formula Ab-2 provided in some embodiments, R¹ is selected from the group consisting of: 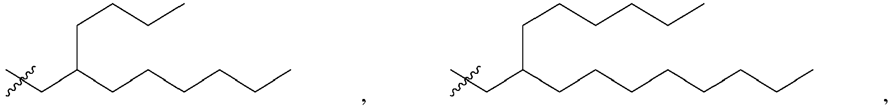 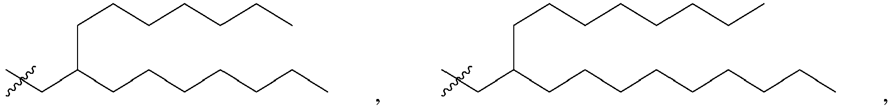 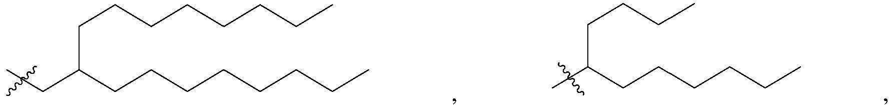 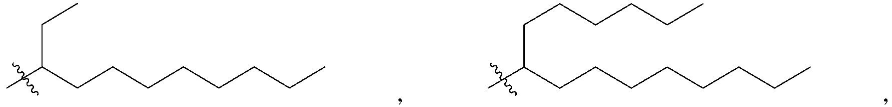 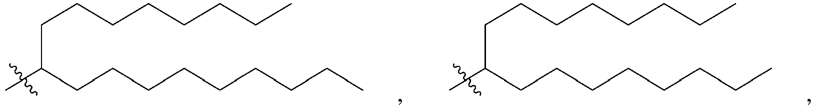 and

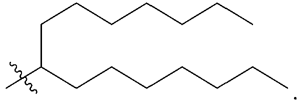

Typical compounds represented by formula A include:

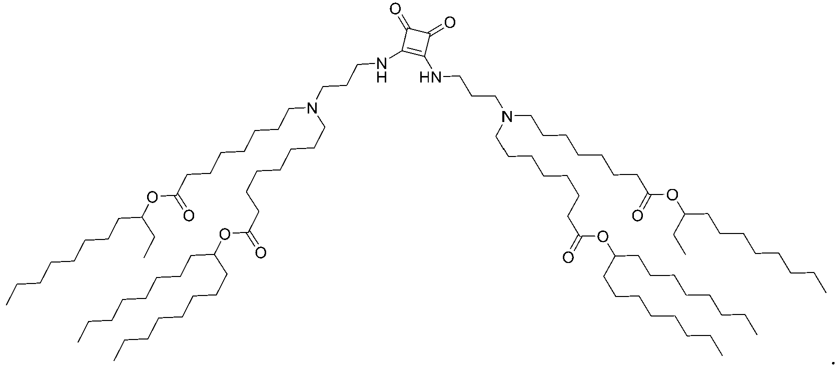

The disclosure further provides an isotopically substituted form of the aforementioned compound or the salt thereof. In some embodiments, the isotopically substituted form is a deuterium-substituted form.

The disclosure further provides a lipid particle comprising the aforementioned compound or the salt thereof or the isotopically substituted form thereof. Further, in some embodiments, the lipid particle further comprises an active agent.

In some embodiments, the active agent is selected from the group consisting of a polynucleotide and a nucleic acid (e.g., a ribonucleic acid or a deoxyribonucleic acid). In some embodiments, the active agent is selected from the group consisting of an mRNA. The disclosure further provides a pharmaceutical composition comprising the aforementioned lipid particle and a pharmaceutically acceptable excipient. In certain embodiments, the pharmaceutical composition comprises 0.01%-99.99% of the pharmaceutically acceptable excipient based on the total weight of the composition. In certain embodiments, the pharmaceutical composition comprises 0.1%-99.9% of the pharmaceutically acceptable excipient. In certain embodiments, the pharmaceutical composition comprises 0.5%-99.5% of the pharmaceutically acceptable excipient. In certain embodiments, the pharmaceutical composition comprises 1%-99% of the pharmaceutically acceptable excipient. In certain embodiments, the pharmaceutical composition comprises 2%-98% of the pharmaceutically acceptable excipient.

In some embodiments, the excipient includes, but is not limited to, a buffer and a lyoprotectant.

In some embodiments, the pharmaceutical composition comprises a buffer, such as tris(hydroxymethyl)aminomethane hydrochloride.

In some embodiments, the pharmaceutical composition comprises a lyoprotectant, such as sucrose.

The disclosure further provides use of the aforementioned compound or the salt thereof, or the isotopically substituted form, or the aforementioned lipid particle, or the aforementioned pharmaceutical composition in the manufacture of a medicament for inducing an immune response in a subject.

The disclosure further provides use of the aforementioned compound or the salt thereof, or the isotopically substituted form, or the aforementioned lipid particle, or the aforementioned pharmaceutical composition in the manufacture of a medicament for preventing and/or treating a disease or disorder associated with polypeptide overexpression.

The disclosure further provides use of the aforementioned compound or the salt thereof, or the isotopically substituted form, or the aforementioned lipid particle, or the aforementioned pharmaceutical composition in the manufacture of a medicament for preventing and/or treating a disease or disorder associated with insufficient polypeptide expression.

In some embodiments, the disease or disorder includes, but is not limited to, cancer, infection, autoimmune diseases, neurodegenerative diseases, and inflammation.

The disclosure further provides a method for preventing and/or treating a disease or disorder associated with induction of an immune response in a subject, comprising administering to the patient the aforementioned compound or the salt thereof or the isotopically substituted form thereof, or the aforementioned lipid particle, or the aforementioned pharmaceutical composition.

The disclosure further provides a method for preventing and/or treating a disease or disorder associated with polypeptide overexpression, comprising administering to the patient the aforementioned compound or the salt thereof or the isotopically substituted form thereof, or the aforementioned lipid particle, or the aforementioned pharmaceutical composition.

The disclosure further provides a method for preventing and/or treating a disease or disorder associated with insufficient polypeptide expression, comprising administering to the patient the aforementioned compound or the salt thereof or the isotopically substituted form thereof, or the aforementioned lipid particle, or the aforementioned pharmaceutical composition.

In another aspect, the disclosure further provides the aforementioned compound or the salt thereof or the isotopically substituted form thereof, or the aforementioned lipid particle, or the aforementioned pharmaceutical composition for use in the prevention and/or treatment of a disease or disorder associated with induction of an immune response in a subject.

The disclosure further provides the aforementioned compound or the salt thereof or the isotopically substituted form thereof, or the aforementioned lipid particle, or the aforementioned pharmaceutical composition for use in the prevention and/or treatment of a disease or disorder associated with polypeptide overexpression.

The disclosure further provides the aforementioned compound or the salt thereof or the isotopically substituted form thereof, or the aforementioned lipid particle, or the aforementioned pharmaceutical composition for use in the prevention and/or treatment of a disease or disorder associated with insufficient polypeptide expression.

The disclosure further provides use of the aforementioned compound or the salt thereof, or the isotopically substituted form, or the aforementioned lipid particle, or the aforementioned pharmaceutical composition in the manufacture of a medicament for preventing and/or treating cancer, infection, autoimmune diseases, neurodegenerative diseases, and inflammation.

The disclosure further provides a method for preventing and/or treating cancer, infection, autoimmune diseases, neurodegenerative diseases, and inflammation, comprising administering to the patient the aforementioned compound or the salt thereof or the isotopically substituted form thereof, or the aforementioned lipid particle, or the aforementioned pharmaceutical composition.

The disclosure further provides the aforementioned compound or the salt thereof or the isotopically substituted form thereof, or the aforementioned lipid particle, or the aforementioned pharmaceutical composition for use in the prevention and/or treatment of cancer, infection, autoimmune diseases, neurodegenerative diseases, and inflammation. The salt of the compound described in the disclosure includes "acid" addition salts and "base" addition salts. For example, salts formed by acid-base reactions with basic groups (amino) are included, and the acid includes organic acids or inorganic acids. In addition, the salt of the compound also includes salts formed by quaternization with basic groups (amino); the quaternizing reagent includes linear or branched chlorinated hydrocarbons. The compounds of the disclosure may have particular geometric or stereoisomeric forms. The disclosure contemplates all such compounds, including cis and trans isomers, (-)- and (+)-enantiomers, (R)- and (S)-enantiomers, diastereomers, (D)-isomer, (L)-isomer, and racemic mixtures, and other mixtures thereof, such as enantiomerically or diastereomerically enriched mixtures, all of which are within the scope of the disclosure. Additional asymmetric carbon atoms may be present in substituents such as an alkyl group. All such isomers and mixtures thereof fall within the scope of the disclosure. The compounds of the disclosure containing asymmetric carbon atoms may be isolated in an optically active pure form or in a racemic form. The optically active pure form may be isolated from a racemic mixture or synthesized using chiral starting materials or chiral reagents.

Optically active (R)- and (S)-isomers, and D- and L-isomers may be prepared by chiral synthesis, using chiral reagents, or other conventional techniques. If one enantiomer of a certain compound of the disclosure is desired, it may be prepared by asymmetric synthesis or derivatization with a chiral auxiliary, wherein the resulting mixture of diastereomers is separated, and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., formation of a carbamate from an amine).

In the chemical structures of the compounds of the disclosure, the bond " 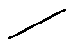 " indicates an unspecified configuration; that is, if chiral isomers exist in the chemical structures, the bond " 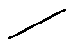 " may be " 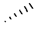 " or " 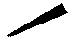 ", or includes both the configurations " 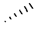 " and " 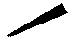 " simultaneously. In the chemical structures of the compounds of the disclosure, the bond " 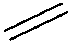 " does not specify a configuration; that is, it may be a Z configuration or an E configuration, or includes both configurations simultaneously.

The compounds and intermediates of the disclosure may also exist in different tautomeric forms, and all such forms are included within the scope of the disclosure. The term "tautomer" or "tautomeric form" refers to structural isomers of different energies that can interconvert via a low-energy barrier. For example, proton tautomers (also known as proton transfer tautomers) include interconversion via proton migration, such as ketoenol, imine-enamine, and lactam-lactim isomerization. An example of lactam-lactim equilibrium is between the following A and B.

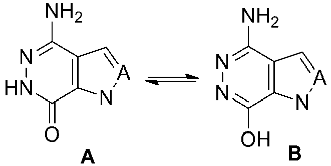

All compounds in the disclosure can be drawn as form A or form B. All tautomeric forms fall within the scope of the disclosure. The names of the compounds do not exclude any tautomers.

The disclosure also includes some isotopically labeled compounds of the disclosure that are identical to those recited herein but have one or more atoms replaced with an atom having an atomic mass or mass number different from the atomic mass or mass number usually found in nature. Examples of isotopes that can be incorporated into the compounds of the disclosure include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds in examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The disclosure also includes various deuterated forms of the compound of formula (I). Each available hydrogen atom linked to a carbon atom may be independently replaced with a deuterium atom. Those skilled in the art can synthesize the deuterated forms of the compound of formula (I) by referring to the relevant literature. Commercially available deuterated starting materials can be used in preparing the deuterated forms of the compound of formula (I), or they can be synthesized using conventional techniques with deuterated reagents, including but not limited to deuterated borane, borane-d₃ in tetrahydrofuran, deuterated lithium aluminum hydride, deuterated iodoethane, deuterated iodomethane, and the like.

"Optionally" or "optional" means that the event or circumstance subsequently described may, but does not necessarily, occur, and this description includes instances where the event or circumstance occurs or does not occur. For example, "C₁₋₆ alkyl optionally substituted with halogen or cyano" means that the halogen or cyano may, but does not necessarily, exist, and this description includes an instance where the alkyl is substituted with halogen or cyano and an instance where the alkyl is not substituted with halogen or cyano.

### Terms and Definitions:

"Pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity. "Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration as acceptable for use in humans or livestock animals.

"Buffer" refers to a buffer that resists changes in pH by the action of its acid-base conjugate components. Examples of buffers that keep pH in an appropriate range include acetate or tris(hydroxymethyl)aminomethane hydrochloride (Tris hydrochloride). "Effective amount", "therapeutically effective amount", or "effective therapeutic amount", as described in the disclosure, includes an amount sufficient to ameliorate or prevent a symptom of a medical disorder or a disorder. An effective amount also means an amount sufficient to allow or facilitate diagnosis. The effective amount for a particular patient or veterinary subject may vary depending on factors such as the disorder to be treated, the general health of the patient, the method, route, and dose of administration, and the severity of side effects. An effective amount may be the maximum dose or administration regimen to avoid significant side effects or toxic effects.

The term "nucleic acid" is a polymer consisting of nucleotides, such as deoxyribonucleotides (DNA) or ribonucleotides (RNA).

The term "oligonucleotide" describes a single-stranded or double-stranded nucleotide polymer that is 2 to 100 nucleotides in length. "Polynucleotide" refers to a single-stranded or double-stranded polymer consisting of nucleotide monomers. In some embodiments, the polynucleotide consists of 100 or more nucleotides.

In some other embodiments, exemplary polynucleotides include, but are not limited to, deoxyribonucleotides (DNAs) and ribonucleic acids (RNAs), including messenger RNAs, RNAi-inducing agents, shRNAs, siRNAs, miRNAs, antisense RNAs, and analogs.

The terms "polypeptide", "peptide", and "protein" are used interchangeably herein to refer to a polymer of amino acid residues. The terms apply to amino acid polymers in which one or more amino acid residues are artificial chemical analogs of corresponding naturally occurring amino acids, as well as to naturally occurring amino acid polymers.

"Alkyl" refers to saturated aliphatic hydrocarbon groups, including linear and branched alkyl groups. In some embodiments, alkyl groups comprise 1-24 carbon atoms and are also known as C₁₋₂₄ alkyl. In some embodiments, alkyl groups comprise 2-24 carbon atoms and are also known as C₂₋₂₄ alkyl. In some embodiments, alkyl groups comprise 4-18 carbon atoms and are also known as C₄-₁₈ alkyl. In some embodiments, alkyl groups comprise 1-12 carbon atoms and are also known as C₁₋₁₂ alkyl. In some embodiments, alkyl groups comprise 1-4 carbon atoms and are also known as C₁₋₄ alkyl. In some embodiments, alkyl groups comprise 10-22 carbon atoms and are also known as C₁₀₋₂₂ alkyl. In some embodiments, alkyl groups comprise 4-22 carbon atoms and are also known as C₄₋₂₂ alkyl.

An alkyl group may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkyl is substituted with one or more groups selected from the group consisting of halogen, hydroxy, oxo, cyano, amino, C₁₋₆ alkyl, C₁₋₆ alkoxy, and 3- to 7-membered cycloalkyl.

"Alkoxy" refers to -O-(alkyl), wherein the alkyl is as defined above. Non-limiting examples of alkoxy groups include methoxy or ethoxy. Alkoxy may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkoxy is substituted with one or more groups selected from the group consisting of halogen, hydroxy, oxo, cyano, amino, C₁₋₆ alkyl, C₁₋₆ alkoxy, and 3-to 7-membered cycloalkyl.

"Alkylamino" refers to -NH(alkyl) or -N(alkyl)₂, wherein the alkyl is as defined above. Exemplary alkylamino groups include C₁₋₆ alkylNH- or di(C₁₋₆ alkyl)N-, such as methylamino or dimethylamino. Alkylamino may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the alkylamino is substituted with one or more groups selected from the group consisting of halogen, hydroxy, oxo, cyano, amino, C₁₋₆ alkyl, C₁₋₆ alkoxy, and 3- to 7-membered cycloalkyl.

"Heteroalkyl" refers to a linear or branched alkyl group that preferably comprises 1 to 14 carbon atoms, more preferably 1 to 12 carbon atoms, and even more preferably 2 to 10 carbon atoms in the chain, wherein one or more carbon atoms are substituted with heteroatoms selected from the group consisting of S, O, and N, including the moiety -O-O- or -S-S-. Exemplary heteroalkyl groups include 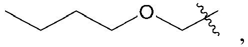 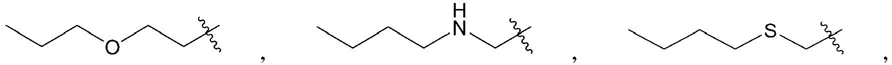 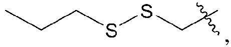 or the like.

Heteroalkyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the heteroalkyl is substituted with one or more groups selected from the group consisting of halogen, hydroxy, oxo, cyano, amino, C₁₋₆ alkyl, C₁₋₆ alkoxy, and 3- to 7-membered cycloalkyl.

"Alkenyl" refers to unsaturated aliphatic hydrocarbon groups, including linear and branched alkenyl groups. In some embodiments, alkenyl groups comprise 2-24 carbon atoms and are also known as C₂₋₂₄ alkenyl. In some embodiments, alkenyl groups comprise 2-12 carbon atoms and are also known as C₂₋₁₂ alkenyl. In some embodiments, alkenyl groups comprise 4-18 carbon atoms and are also known as C₄-₁₈ alkenyl. In some embodiments, alkenyl groups comprise 1-4 carbon atoms and are also known as C₁₋₄ alkenyl. In some embodiments, alkenyl groups comprise 10-22 carbon atoms and are also known as C₁₀₋₂₂ alkenyl. In some embodiments, alkenyl groups comprise 4-22 carbon atoms and are also known as C₄₋₂₂ alkenyl. Exemplary alkenyl groups include ethenyl, propenyl, n-butenyl, isobutenyl, 3-methylbut-2-enyl, n-pentenyl, heptenyl, octenyl, cyclohexyl-butenyl, and decenyl.

Alkenyl may be unsubstituted or substituted with one or more groups selected from the group consisting of halogen, hydroxy, oxo, cyano, amino, C₁₋₆ alkyl, C₁₋₆ alkoxy, and 3-to 7-membered cycloalkyl. In some embodiments, the alkenyl is substituted with one or more groups selected from the group consisting of halogen, hydroxy, oxo, cyano, amino, C₁₋₆ alkyl, C₁₋₆ alkoxy, and 3- to 7-membered cycloalkyl.

For the definition of "heteroalkenyl", see the definition of "heteroalkyl". Heteroalkenyl refers to a linear or branched alkenyl group in which one or more carbon atoms are substituted with heteroatoms selected from the group consisting of S, O, and N, including the moiety -O-O- or -S-S-.

"Monovalent group" refers to a group formed by "formally" eliminating a monovalent atom or group from a compound. "-ylene" refers to a group formed by "formally" eliminating two monovalent atoms or groups of atoms or one divalent atom or group of atoms from a compound.

The term "alkylene" refers to the remainder of an alkane molecule after 2 hydrogen atoms are removed from the alkane molecule. In some embodiments, alkylene groups comprise 1-12 carbon atoms and are also known as C₁₋₁₂ alkylene. In some embodiments, alkylene groups comprise 3-8 carbon atoms and are also known as C₃₋₈ alkylene. In some embodiments, alkylene groups comprise 1-4 carbon atoms and are also known as C₁₋₄ alkylene. In some embodiments, alkyl groups comprise 10-22 carbon atoms and are also known as C₁₀₋₂₂ alkylene. In some embodiments, alkyl groups comprise 4-22 carbon atoms and are also known as C₄₋₂₂ alkylene.

Alkylene may be unsubstituted or substituted with one or more groups selected from the group consisting of halogen, hydroxy, oxo, cyano, amino, C₁₋₆ alkyl, C₁₋₆ alkoxy, and 3-to 7-membered cycloalkyl. In some embodiments, the alkylene is substituted with one or more groups selected from the group consisting of halogen, hydroxy, oxo, cyano, amino, C₁₋₆ alkyl, C₁₋₆ alkoxy, and 3- to 7-membered cycloalkyl.

The term "heteroalkylene" refers to the remainder of a heteroalkane molecule after 2 hydrogen atoms are removed from the heteroalkane molecule. In some embodiments, heteroalkylene comprises 1-14 carbon atoms and is also known as C₁₋₄ heteroalkylene. In some embodiments, heteroalkylene comprises 1-12 carbon atoms and is also known as C₁₋₁₂ heteroalkylene. In some embodiments, heteroalkylene comprises 2-10 carbon atoms and is also known as C₂₋₁₀ heteroalkylene. In some embodiments, heteroalkylene comprises 2-9 carbon atoms and is also known as C₂₋₉ heteroalkylene. In some other embodiments, one or more carbon atoms in the chain of heteroalkyl are substituted with a heteroatom selected from the group consisting of S, O, and N. Exemplary heteroalkylene groups include 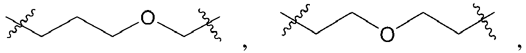 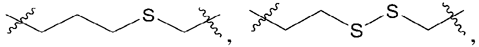 or the like.

Heteroalkylene may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the heteroalkylene is substituted with one or more groups selected from the group consisting of halogen, hydroxy, oxo, cyano, amino, C₁₋₆ alkyl, C₁₋₆ alkoxy, and 3- to 7-membered cycloalkyl.

Likewise, the definitions of "alkyleneoxy" and "alkenylene" are the same as the definition of "alkylene".

The term "cycloalkyl" refers to a saturated or partially unsaturated, monocyclic or polycyclic hydrocarbon substituent, and preferably, the cycloalkyl ring contains 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, etc. Polycyclic cycloalkyl groups include spiro-ring, fused-ring, and bridged-ring cycloalkyl groups. Cycloalkyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the cycloalkyl is substituted with one or more groups selected from the group consisting of halogen, hydroxy, oxo, cyano, amino, C₁₋₆ alkyl, C₁₋₆ alkoxy, and 3- to 7-membered cycloalkyl.

"Cycloalkenyl" refers to an unsaturated, monocyclic or polycyclic hydrocarbon substituent containing one or more carbon-carbon double bonds. Preferably, cycloalkenyl comprises 3 to 8 carbon atoms, e.g., C3-C8 cycloalkenyl. In addition, cycloalkenyl may be fused to aryl or heteroaryl. Exemplary cycloalkenyl groups include, but are not limited to: 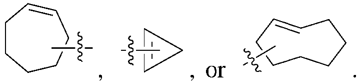 In addition, cycloalkenyl may be unsubstituted or substituted with one or more groups selected from the group consisting of halogen, hydroxy, oxo, cyano, amino, C₁₋₆ alkyl, C₁₋₆ alkoxy, and 3- to 7-membered cycloalkyl. In some embodiments, the cycloalkenyl is substituted with one or more groups selected from the group consisting of halogen, hydroxy, oxo, cyano, amino, C₁₋₆ alkyl, C₁₋₆ alkoxy, and 3- to 7-membered cycloalkyl.

The terms "branched alkyl" and "branched alkenyl" refer to alkyl and alkenyl groups in which one carbon atom is linked to at least 2 other carbon atoms, and a cyclic group, such as a spiro ring, is not formed. Examples of C₆ alkyl groups include 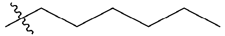 (linear) or 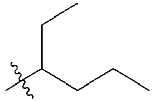 (branched).

Likewise, the definitions of "alkyleneoxy", "alkenylene", "cycloalkylene", and "cycloalkenylene" are the same as the definition of "alkylene".

The term "hydroxy" refers to the -OH group.

The term "halogen" refers to fluorine, chlorine, bromine, or iodine.

The term "amino" refers to -NH₂.

The term "cyano" refers to -CN.

The term "oxo" refers to the =O substituent.

"Substituted" means that one or more, preferably up to 5, and more preferably 1 to 3, hydrogen atoms in the group are independently substituted with a corresponding number of substituents. It goes without saying that a substituent is only in its possible chemical position, and those skilled in the art will be able to determine (experimentally or theoretically) possible or impossible substitutions without undue effort.

### BRIEF DESCRIPTION OF THE DRAWINGS

FIG. 1: the fluorescence intensities of mice 24 h after injection of the mice with lipid nanoparticles of luciferase mRNA via the tail vein.
FIG. 2: the fluorescence intensities of mice 48 h after injection of the mice with lipid nanoparticles of luciferase mRNA via the tail vein.

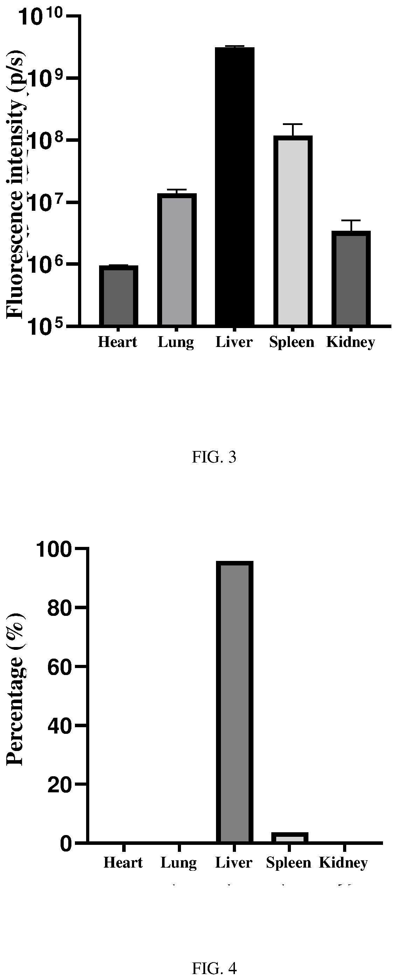

FIG. 3: the fluorescence intensities of various organ tissues of mice injected with lipid nanoparticles of luciferase mRNA via the tail vein.
FIG. 4: the fluorescence intensity proportions of various organ tissues of mice injected with lipid nanoparticles of luciferase mRNA via the tail vein.
FIG. 5: the fluorescence intensities of the muscles at the injection sites of mice 24 h after intramuscular injection of the mice with lipid nanoparticles of luciferase mRNA.
FIG. 6: the protein expression levels in the muscles at the injection sites of mice 6 h after intramuscular injection of the mice with lipid nanoparticles of hepatocyte growth factor mRNA.
FIG. 7: the concentration of interleukin 6 (IL-6) in the muscles at the injection sites of mice 6 h after intramuscular injection of the mice with lipid nanoparticles of hepatocyte growth factor mRNA.
FIG. 8: the protein expression levels after delivery of mRNA into HEK293 cells by lipid nanoparticles.
FIG. 9: the protein expression levels after delivery of mRNA into HuH-7 cells by lipid nanoparticles.
FIG. 10: the fluorescence intensities of mice 24 h after injection of the mice with lipid nanoparticles of luciferase mRNA via the tail vein.
FIG. 11: the fluorescence intensities of mice 48 h after injection of the mice with lipid nanoparticles of luciferase mRNA via the tail vein.

### DETAILED DESCRIPTION

The disclosure is further described below with reference to examples, but these examples are not intended to limit the scope of the disclosure.

Experimental methods without specific conditions indicated in the examples of the disclosure were generally conducted under conventional conditions, or conditions recommended by the manufacturers of the starting materials or commercial products. Reagents without specific sources indicated were commercially available conventional reagents.

The structures of the compounds were determined by nuclear magnetic resonance (NMR) spectroscopy or/and mass spectrometry (MS). The NMR shifts (δ) are given in 10⁻⁶ (ppm). The NMR analyses were performed on a Bruker AVANCE-400 nuclear magnetic resonance instrument, with dimethyl sulfoxide-D6 (DMSO-*d₆*), chloroform-D (CDCl₃), or methanol-D4 (methanol-*d₄*) as the solvent and tetramethylsilane (TMS) as the internal standard.

The HPLC analyses were performed using an Agilent 1100 high pressure liquid chromatograph, a GAS15B DAD ultraviolet detector, and a Water Vbridge C18 150 × 4.6 mm 5 µm chromatography column.

The MS analyses were performed using an Agilent 6120 triple quadrupole mass spectrometer, a G1315D DAD detector, and a Waters Xbridge C18 4.6 × 50 mm, 5 µm chromatography column, with scanning performed in positive/negative ion mode with a mass scan range of 80-1200.

The thin-layer chromatography silica gel plates used were Yantai Huanghai HSGF254 silica gel plates. The silica gel plates used in the thin-layer chromatography (TLC) analyses had a layer thickness of 0.2 mm ± 0.03 mm, and those used in the thin-layer chromatography separation and purification had a layer thickness of 0.4 mm-0.5 mm. The flash column purification system used was Combiflash Rf150 (TELEDYNE ISCO) or Isolara one (Biotage).

In the normal-phase column chromatography, a 200-300 mesh or 300-400 mesh Yantai Huanghai silica gel was generally used as the carrier, or a Changzhou Santai pre-fill ultrapure normal-phase silica gel column (40-63 µm, 60 g, 24 g, 40 g, 120 g, or other specifications) was used.

The known starting materials in the disclosure may be synthesized by using or following methods known in the art, or may be purchased from Shanghai Titan Scientific, ABCR GmbH & Co. KG, Acros Organics, Aldrich Chemical Company, Accela ChemBio Inc., and Bide Pharmatech, among others.

In the examples, the reactions can all be performed in a nitrogen atmosphere unless otherwise specified.

The nitrogen atmosphere means that the reaction flask is connected to a balloon containing about 1 L of nitrogen.

The hydrogen atmosphere means that the reaction flask is connected to a balloon containing about 1 L of hydrogen.

The hydrogen gas was prepared by a QPH-1L hydrogen generator from Shanghai Quan Pu Scientific Instruments Inc.

The nitrogen atmosphere or the hydrogenation atmosphere was generally formed by 3 cycles of vacuumization and nitrogen or hydrogen filling.

In the examples, the solutions were aqueous solutions unless otherwise specified.

In the examples, the reaction temperature was room temperature, i.e., 20 °C-30 °C, unless otherwise specified.

The monitoring of the reaction progress in the examples was performed using thin-layer chromatography (TLC). The developing solvents used in the reactions, the eluent systems used in the column chromatography purification of the compounds, the developing solvent systems used in the thin-layer chromatography, and the volume ratio of the solvents were adjusted depending on the polarity of the compounds, or a small amount of basic or acidic reagents, such as triethylamine and acetic acid, could be added for adjustment.

### Example 1: Synthesis of Compound 1

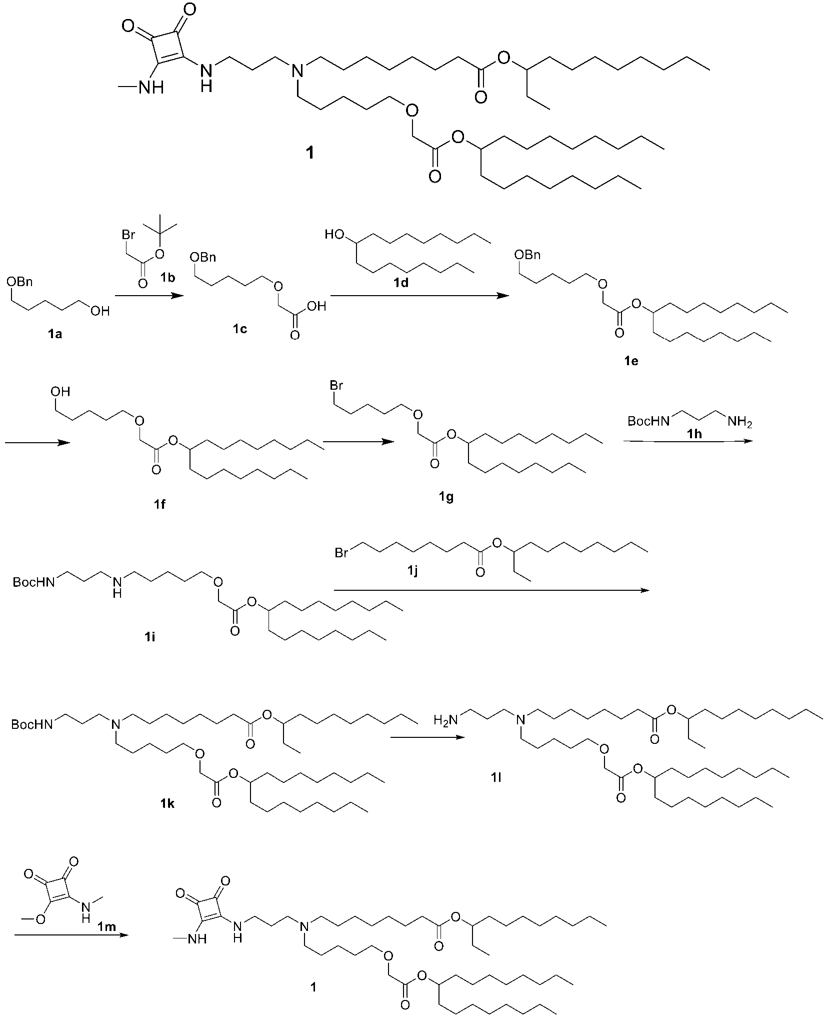

### Step 1:

Water (46.8 mL) was added to a 500 mL three-necked flask. After the temperature was lowered to below 5 °C using an ice-water bath, sodium hydroxide (46.8 g, 1170 mmol) was slowly added. After sodium hydroxide was completely dissolved, toluene (46.8 mL) and compound 1a (9.71 g, 50.0 mmol, purchased from Adamas) were added, and the mixture was stirred at room temperature for 1 h. The mixture was then cooled to below 5 °C in an ice-water bath, and compound 1b (34.1 g, 175 mmol, purchased from Adamas) was slowly added dropwise. The mixture was heated to 25 °C in an oil bath and stirred until the reaction was complete. The reaction was cooled. Water (200 mL) was added, and extraction was performed with methyl tert-butyl ether (100 mL × 3). The aqueous phase was cooled to below 5 °C, and concentrated hydrochloric acid was added until the pH value was about 3. Extraction was then performed with DCM (100 mL × 5). The DCM phases were combined, washed with saturated brine, dried over anhydrous sodium sulfate, and concentrated under reduced pressure, and the residue was purified by column chromatography (EA/PE = 0-100%) to give compound 1c (11.97 g, yield: 94%).

¹H NMR (400 MHz, CDCl₃): *δ* 8.50 (brs, 1H), 7.36-7.26 (m, 5H), 4.51 (s, 2H), 4.13-4.10 (m,2H), 3.56 (t, 2H), 3.48 (t, 2H), 1.67-1.61 (m, 4H), 1.50-1.43 (m, 2H).

### Step 2:

Compound 1c (21.7 g, 86.0 mmol) was weighed into a reaction flask, and DCM (434 mL), compound 1d (19.9 g, 77.4 mmol, prepared according to the method described in Tetrahedron, 2011, vol. 67, # 2, p. 303-311*),* DMAP (10.5 g, 30.1 mmol), and EDCI (19.8 g, 103 mmol) were sequentially added. The mixture was left to react overnight at room temperature. TLC monitoring showed that the reaction had been complete. Water (500 mL) was added to the reaction mixture, and the aqueous phase was isolated and extracted with DCM (300 mL). The organic phases were combined and then washed with a saturated aqueous sodium bicarbonate solution, washed with saturated brine, dried over anhydrous sodium sulfate, and concentrated under reduced pressure, and the residue was purified by column chromatography (EA/PE = 0-5%) to give compound le (41.5 g, yield: 98%).

¹H NMR (400 MHz, CDCl₃): *δ* 7.34-7.33 (m, 4H), 7.28-7.27 (m, 1H), 4.97-4.95 (m, 1H), 4.50 (s, 2H), 4.04 (s, 2H), 3.54-3.46 (m, 4H), 1.69-1.62 (m, 4H), 1.58-1.43 (m, 2H), 1.25 (s, 26H), 0.89-0.83 (m, 8H).

### Step 3:

Compound le (41.5 g, 84.3 mmol) was weighed out and dissolved in THF (208 mL), and Pd(OH)₂/C (4.15 g, 20%) was added. Hydrogen was introduced into the system using a double-layer hydrogen balloon, and the mixture was stirred at room temperature. TLC monitoring showed that the reaction had been complete. The reaction mixture was filtered through diatomaceous earth under vacuum, and the filter cake was washed with EA (100 mL × 3). After concentration under reduced pressure, the residue was purified by column chromatography (EA/PE = 50%) to give compound 1f (27.0 g, yield: 80%).

¹H NMR (400 MHz, CDCl₃): *δ* 4.98-4.93 (m, 1H), 4.05 (s, 2H), 3.66 (t, 2H), 3.54 (t, 2H), 1.70-1.58 (m, 4H), 1.53-1.43(m, 4H), 1.25 (s, 26H), 0.89-0.86 (m, 6H).

### Step 4:

Compound 1f (6.01 g, 15.0 mmol) was weighed out and dissolved in DCM (60 mL), and carbon tetrabromide (7.46 g, 22.5 mmol) was added under ice-bath conditions. After 10 min of stirring, triphenylphosphine (5.51 g, 21.0 mmol) was added. After the addition, the mixture was stirred at room temperature. TLC monitoring showed that the reaction had been complete. The reaction mixture was purified by column chromatography (EA/PE = 0-5%) to give compound 1g (6.75 g, yield: 97%).

¹H NMR (400 MHz, CDCl₃): *δ* 4.99-4.93 (m, 1H), 4.05 (s, 2H), 3.54 (t, 2H), 3.41 (t, 2H), 1.94-1.86 (m, 2H), 1.70-1.63 (m, 2H), 1.58-1.52 (m, 5H), 1.26 (s, 25H), 0.88 (t, 6H).

### Step 5:

Compound 1g (1.0 g, 2.2 mmol) and compound 1h (1.9 g, 11.0 mmol, purchased from Energy Chemical) were dissolved in 20 mL of absolute ethanol, and the solution was heated to an internal temperature of 60 °C and left to react for 16 h in a nitrogen atmosphere. The reaction mixture was cooled to room temperature and concentrated under reduced pressure, and the residue was purified by column chromatography (DCM:MeOH = 10:1 to 5:1) to give compound 1i (1.0 g, yield: 83%).

¹H NMR (400 MHz, CDCl₃): *δ* 5.13 (br s 1H), 4.99-4.93 (m, 1H), 4.04 (s, 2H), 3.53 (t, 2H), 3.25-3.16 (m, 2H), 2.69 (t, 2H), 2.63 (t, 2H), 1.71-1.61 (m, 4H), 1.57-1.40 (m, 18H), 1.33-1.19 (m, 24H), 0.92-0.84 (m, 6H).

### Step 6:

Compound 1i (0.487 g, 0.87 mmol), compound 1j (0.300 g, 0.795 mmol, prepared according to the method described in Patent Application No. WO2018/170306), potassium iodide (0.145 g, 0.87 mmol), and potassium carbonate (0.438 g, 3.018 mmol) were added to a 25 mL flask, and cyclopentyl methyl ether and anhydrous acetonitrile (5 mL each) were added. The system was heated in a nitrogen atmosphere until the reaction was substantially complete. The reaction mixture was cooled to room temperature, filtered, and concentrated, and the residue was purified by column chromatography (PE:EA = 10:1) to give compound 1k (0.5 g, yield: 74%).

¹H NMR (400 MHz, CDCl₃): *δ* 5.60 (br s 1H), 4.99-4.93 (m, 1H), 4.84-4.78 (m, 1H), 4.04 (s, 2H), 3.53 (t, 2H), 3.21-3.12 (m, 2H), 2.51-2.25 (m, 8H), 1.67-1.25 (m, 71H), 0.89-0.83 (m, 12H).

### Step 7:

Compound 1k (0.5 g, 0.586 mmol) was dissolved in anhydrous dichloromethane (6.0 mL), and trifluoroacetic acid (2.0 mL) was added at room temperature. The mixture was left to react for 4 h. The reaction mixture was concentrated under reduced pressure, and the residue was purified by column chromatography (water:acetonitrile = 35%-95%) to give compound 11 (370 mg, yield: 84%).

¹H NMR (400 MHz, CDCl₃): *δ* 4.99-4.93 (m, 1H), 4.84-4.78 (m, 1H), 4.04 (s, 2H), 3.51 (t, 2H), 2.72 (t, 2H), 2.46-2.35 (m, 6H), 2.29 (t, 2H), 1.67-1.25 (m, 62H), 0.89-0.83 (m, 12H).

### Step 8:

Compound 11 (318 mg, 0.423 mmol) and compound 1m (59.6 mg, 0.423 mmol, prepared according to the method described in Chemistry Letters, 2007, vol. 36, # 8, p. 1012-1013*)* were added to a flask, and absolute ethanol (20 mL) was added. The system was left to react at 25-35 °C for 18 h in a nitrogen atmosphere. The reaction mixture was concentrated under reduced pressure, and the residue was purified by column chromatography (water:acetonitrile = 35%-100%) to give compound 1 (290 mg, yield: 80%).

¹H NMR (400 MHz, CDCl₃): *δ* 4.96-4.92 (m, 1H), 4.82-4.79 (m, 1H), 4.05 (s, 2H), 3.67 (br s, 2H), 3.53 (t, 2H), 3.27 (d, 3H), 2.54 (t, 2H), 2.46-2.40 (m, 4H), 2.29 (t, 2H), 1.79-1.29 (m, 62H), 0.89-0.83 (m, 12H).

### Example 2: Synthesis of Compound 2

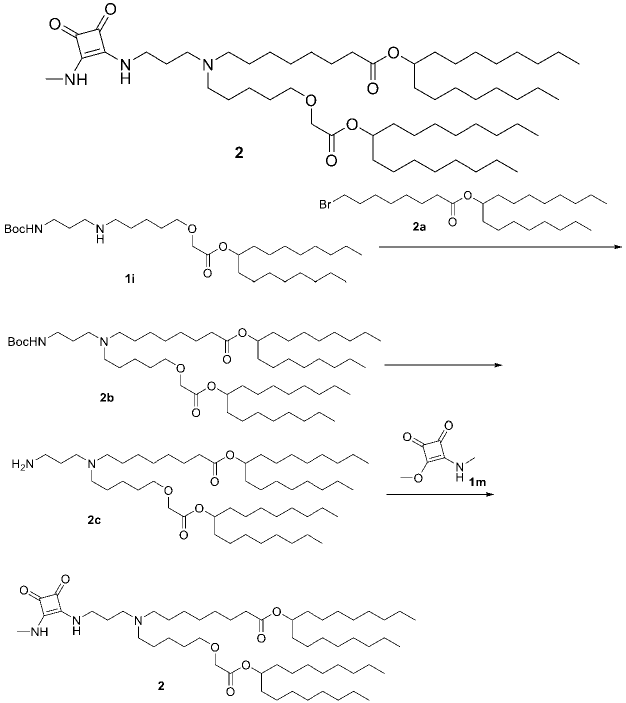

### Step 1:

Compound 1i (1.0 g, 1.8 mmol) and compound 2a (0.835 g, 1.8 mmol, prepared using the method described in Patent Application No. WO2017/201333) were added to a flask. Potassium carbonate (0.996 g, 7.21 mmol) and potassium iodide (0.328 g, 1.98 mmol) were added, and the solvents anhydrous acetonitrile and cyclopentyl methyl ether (10 mL each) were added. The mixture was heated in a nitrogen atmosphere until the reaction was substantially complete. The reaction mixture was cooled to room temperature, filtered, and concentrated, and the residue was purified by column chromatography (PE:EA = 10:1) to give compound 2b (1.05 g, yield: 62%).

¹H NMR (400 MHz, CDCl₃): *δ* 5.61 (br s, 1H), 4.99-4.93 (m, 1H), 4.89-4.83 (m, 1H), 4.04 (s, 2H), 3.52 (t, 2H), 3.19-3.14 (m, 2H), 2.46-2.26 (m, 8H), 1.69-1.18 (m, 83H), 0.89-0.86 (m, 12H).

### Step 2:

Compound 2b (1.05 g, 1.12 mmol) was added to a flask, and anhydrous dichloromethane (8 mL) was added. In a nitrogen atmosphere, a 4 M HCl/dioxane solution (4 mL) was added at room temperature, and the mixture was left to react for 4 h. The reaction mixture was concentrated under reduced pressure to give a crude product, and the crude product was dissolved in 50 mL of dichloromethane. The resulting solution was washed twice with a saturated aqueous sodium carbonate solution, washed once with 20 mL of a 0.1 N aqueous NaOH solution, dried over anhydrous sodium sulfate, filtered, and concentrated, and the residue was purified by column chromatography (acetonitrile:methanol = 0-100%) to give compound 2c (0.85 g, yield: 91%).

¹H NMR (400 MHz, CDCl₃): *δ* 4.99-4.93 (m, 1H), 4.89-4.83 (m, 1H), 4.04 (s, 2H), 3.52 (t, 2H), 2.72 (t, 2H), 2.46-2.35 (m, 6H), 2.27 (t, 2H), 1.69-1.18 (m, 74H), 0.89-0.82 (m, 12H).

### Step 3:

Compound 2c (0.25 g, 0.298 mmol) was added to a flask. Compound 1m (0.042 g, 0.298 mmol) was added, and absolute ethanol (5 mL) was added. The mixture was left to react at room temperature for 16 h. The reaction mixture was concentrated under reduced pressure, and the residue was purified by column chromatography (acetonitrile:methanol = 0-100%) to give compound 2 (0.25 g, yield: 89%).

¹H NMR (400 MHz, CDCl₃): *δ* 8.69 (br s, 1H), 8.22 (br s, 1H), 4.96-4.90 (m, 1H), 4.89-4.82 (m, 1H), 4.03 (s, 2H), 3.76-3.67 (m, 2H), 3.53 (t, 2H), 3.31 (s, 3H), 3.28-3.20 (m, 2H), 3.16-2.99 (m, 4H), 2.28 (t, 2H), 2.15-2.04 (m, 2H), 1.83-1.17 (m, 72H), 0.91-0.82 (m, 12H).

### Example 3: Synthesis of Compound 3

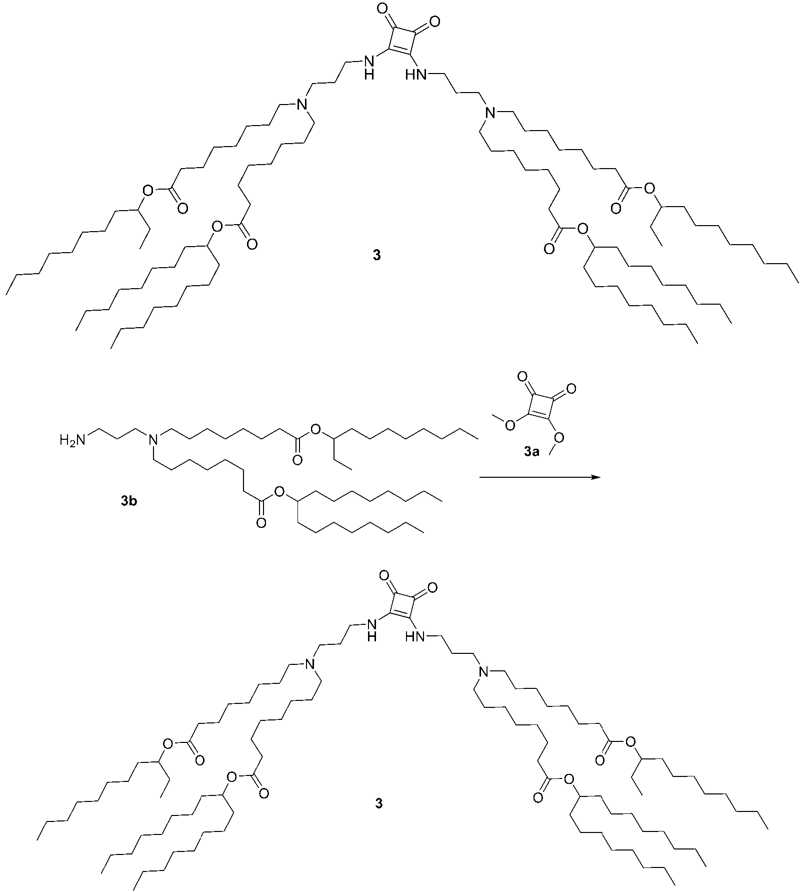

Compound 3b (0.4 g, 0.532 mmol, prepared using the method described in Patent Application No. WO2018/170306) and compound 3a (37.8 mg, 0.266 mmol, prepared using the method described in Organic Syntheses, 1999, vol. 76, p. 189-189*)* were added to a flask, and absolute ethanol (18 mL) was added. The mixture was left to react at room temperature for 16 h in a nitrogen atmosphere. The reaction mixture was concentrated under reduced pressure, and the residue was purified by column chromatography (acetonitrile:methanol = 0-100%) to give compound 3 (0.19 g, yield: 45%).

¹H NMR (400 MHz, CDCl₃): *δ* 7.11 (br s, 2H), 4.89-4.77 (m, 4H), 3.73-3.58 (m, 4H), 2.61-2.52 (m, 4H), 2.46-2.36 (m, 8H), 2.32-2.24 (m, 8H), 1.78-1.17 (m, 132H), 0.93-0.79 (m, 24H).

### Example 4: Synthesis of Compound 4

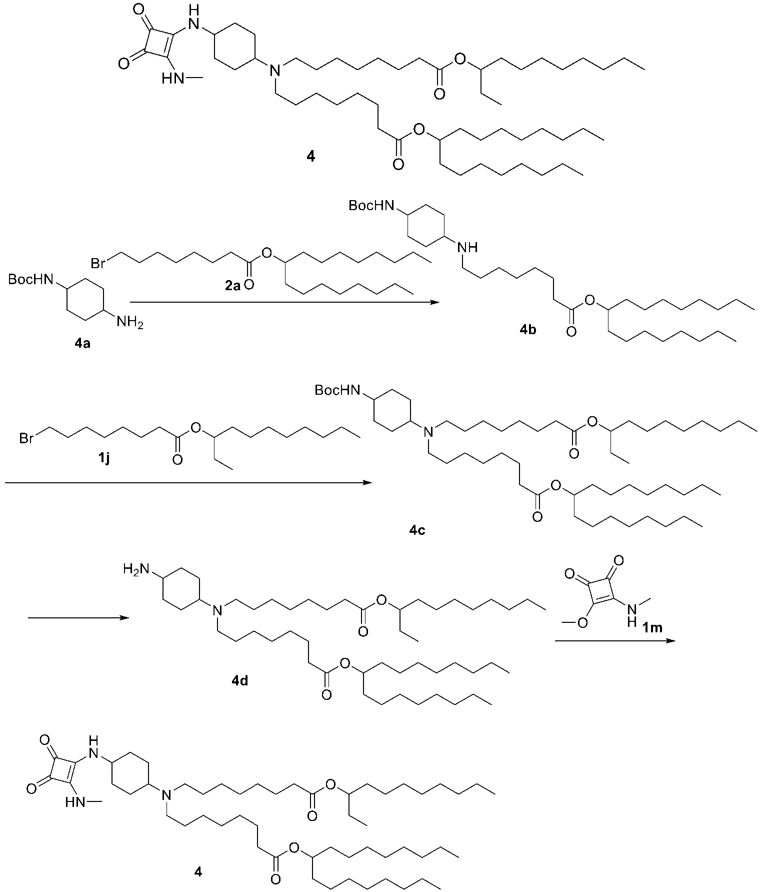

### Step 1:

Compound 4a (2.3 g, 10.1 mmol, purchased from Leyan) and compound 2a (1.0 g, 2.1 mmol) were added to a flask, and absolute ethanol (60 mL) was added. The mixture was heated and left to react for 18 h in a nitrogen atmosphere, and the heating was stopped. The reaction mixture was cooled to room temperature and concentrated, and the residue was purified by column chromatography (DCM:MeOH = 10:1) to give compound 4b (0.85 g, yield: 65%).

¹H NMR (400 MHz, CDCl₃): *δ* 4.89-4.83 (m, 1H), 4.71-4.33 (m, 1H), 3.71-3.38 (m, 1H), 2.62-2.41 (m, 3H), 2.29-2.25 (m, 2H), 2.03-1.93 (m, 2H), 1.70-1.10 (m, 54H), 0.89-0.84 (m, 6H).

### Step 2:

Compound 4b (400 mg, 0.67 mmol) and compound 1j (254 mg, 0.67 mmol) were added to a flask. Potassium carbonate (371.5 mg, 2.69 mmol) and potassium iodide (123.0 mg, 0.74 mmol) were added, and the solvents anhydrous acetonitrile and cyclopentyl methyl ether (5 mL each) were added. The mixture was heated in a nitrogen atmosphere until the reaction was substantially complete. The reaction mixture was cooled to room temperature, filtered, and concentrated, and the residue was purified by column chromatography (PE:EA = 10:1) to give compound 4c (211 mg, yield: 35%).

MS m/z (ESI): 891.7 [M+H]⁺.

### Step 3:

Compound 4c (0.211 g, 0.237 mmol) was dissolved in anhydrous dichloromethane (1.5 mL), and trifluoroacetic acid (1.5 mL) was added. The mixture was left to react at room temperature for 4 h. The reaction mixture was concentrated under reduced pressure, and the residue was purified by column chromatography (water:acetonitrile = 35%-95%) to give compound 4d (126 mg, yield: 67%).

¹H NMR (400 MHz, CDCl₃): *δ* 4.89-4.78 (m, 2H), 2.47-2.34(m, 4 H), 2.31-2.24 (m, 4H), 1.68-1.18 (m, 76H), 0.92-0.83 (m, 12H).

### Step 4:

Compound 4d (81 mg, 0.102 mmol) and compound 1m (29.2 mg, 0.206 mmol) were added to a flask, and absolute ethanol (5 mL) was added. The system was left to react at 25-35 °C in a nitrogen atmosphere. TLC monitoring showed that the reaction had been complete. The reaction mixture was concentrated under reduced pressure, and the residue was purified by column chromatography (water:acetonitrile = 35%-95%) to give compound 4 (88 mg, yield: 95%).

¹H NMR (400 MHz, CDCl₃): *δ* 4.89-4.77 (m, 2H), 2.37-2.28 (m, 3 H), 2.56-2.24 (m, 8 H), 2.15-2.06 (m, 1H), 1.84-1.18 (m, 75H), 0.93-0.81 (m, 12H).

### Example 5

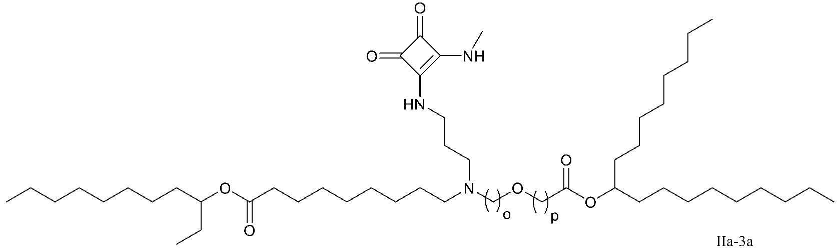

| No. | | Preparation method |
|---|---|---|
| Compound 5 | o = 3, p = 3 | Obtained by referring to the method in Example 1, using 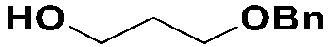 in place of 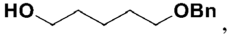 and |
| | | using 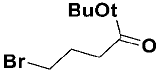 in place of 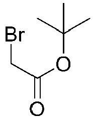 |
| Compound 6 | o = 2, p = 4 | Obtained by referring to the method in Example 1, using 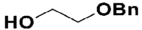 in place of 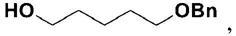 and using 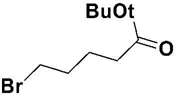 in place of 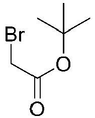 |

### Compound 5:

MS (ESI): m/z 862.68 [M+H]⁺.

¹H NMR (400 MHz, CDCl₃): *δ* 4.78-4.85 (m, 2H), 3.66-3.68 (m, 2H), 3.42-3.47 (m, 4H), 3.27-3.29 (m, 3H), 2.49-2.54 (m, 4H), 2.36-2.40 (m, 4H), 2.27-2.31 (m, 2H), 1.20-1.80 (m, 62H), 0.85-0.89 (m,12H).

### Compound 6:

MS (ESI): m/z 862.68 [M+H]⁺.

¹H NMR (400 MHz, CDCl₃):6 4.79-4.86 (m, 2H), 3.28-3.58 (m, 9H), 2.60-2.62 (m, 4H), 2.42-2.46(m, 2H), 2.28-2.34(m, 4H),1.20-1.80 (m, 62H), 0.86-0.89 (m,12H).

### Comparative Example 1

Obtained by referring to the method in US10392341

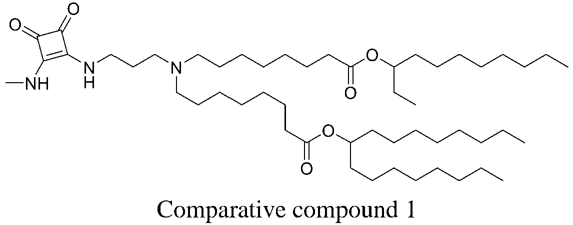

### Comparative Example 2

Obtained by referring to the method in WO2023125738

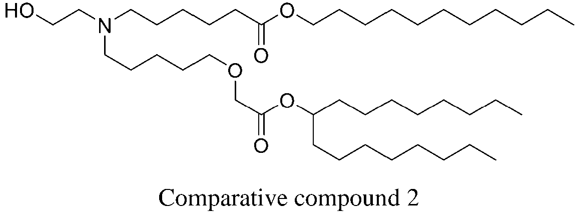

### Comparative Example 3

Obtained by referring to the method in US9868692

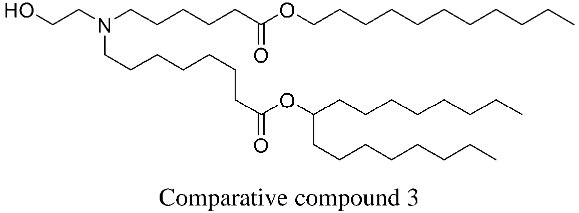

### Example 6: Preparation of Lipid Nanoparticle Compositions

### 1.1. Preparation method

Compounds 1-6 and comparative compounds 1-3 were each dissolved in an ethanol solution, and each of the resulting solutions was mixed with solutions of DSPC, cholesterol, and DMG-PEG in ethanol in a molar ratio of 50:10:38.5:1.5 to prepare an ethanol lipid solution. Luciferase mRNA (GenBank accession No. MN728548.1) was dissolved in a 50 mM acetic acid buffer (pH 5) to prepare an aqueous mRNA solution (0.2 mg/mL). Each of the ethanol lipid solutions was mixed with the aqueous mRNA solution by microfluidics, with the volume ratio of the ethanol phase to the aqueous phase being 1:3 and the weight ratio of total lipids to mRNA being about 20:1, to prepare lipid nanoparticles. Ethanol was removed by dialysis against a 20 mM Tris solution (pH 7.5). Finally, the lipid nanoparticles were exchanged into a solution containing 20 mM Tris, pH 7.5, and 8% sucrose, and the resulting solutions were cryopreserved to obtain mRNA-encapsulating lipid nanoparticle compositions.

### 1.2. Characterization of lipid nanoparticle compositions

The size and polydispersity index (PDI) of the lipid nanoparticles were determined using a Malvern Zetasizer Pro in a 173° backscatter detection mode and dynamic light scattering. The liposome encapsulation efficiency was determined using a Quant-iT RiboGreen RNA assay kit.

**Table 1**

| No. | Size (nm) | PDI | Encapsulation efficiency (%) |
|---|---|---|---|
| Compound 1 | 69.6 | 0.08 | 98 |
| Compound 2 | 63.3 | 0.10 | 99 |
| Compound 3 | 102.4 | 0.04 | 96 |
| Compound 4 | 64.2 | 0.10 | 99 |
| Compound 5 | 64.4 | 0.06 | 98 |
| Compound 6 | 71.9 | 0.16 | 98 |
| Comparative compound 1 | 64.8 | 0.13 | 98 |
| Comparative compound 2 | 90.3 | 0.17 | 92 |
| Comparative compound 3 | 96.0 | 0.03 | 94 |

### Test Example 1: Assessment of mRNA Delivery Efficiency and Tissue Distribution of In Vivo Tail Vein Injection of Lipid Nanoparticle Compositions

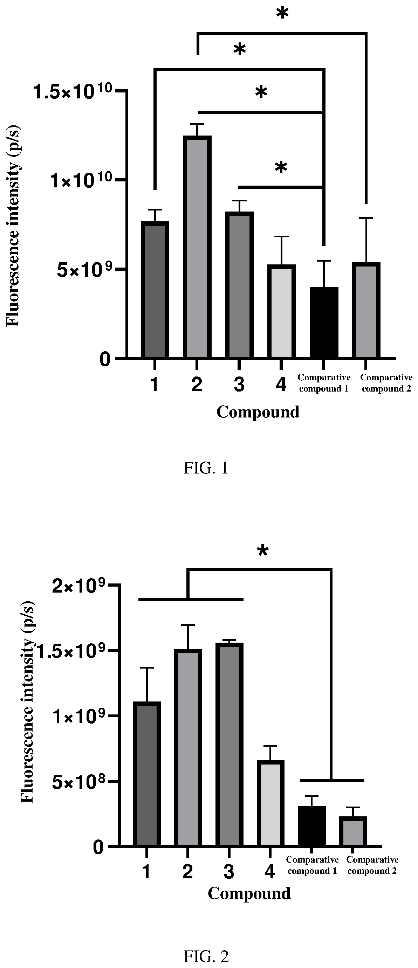

Lipid nanoparticle compositions encapsulating a luciferase-expressing mRNA (GenBank accession No. MN728548.1) were administered to female Balb/c mice aged 6-8 weeks by tail vein injection at a dose of 0.5 mg/kg. Twenty-four hours and forty-eight hours after administration, the luciferase substrate was injected intraperitoneally into each mouse. Fluorescence photos of tissues were taken using an IVIS small-animal optical in vivo imaging system (PerkinElme), and the fluorescence intensity was measured. The fluorescence intensity indicates the level of luciferase protein expression, i.e., the *in vivo* mRNA delivery efficiency of the lipid nanoparticles. The 24-hour mice were dissected. Their hearts, livers, spleens, lungs, and kidneys were collected and photographed using an IVIS small-animal optical in vivo imaging system, and the fluorescence intensity of each tissue was measured. For the lipid nanoparticle composition corresponding to each compound, at least 3 biological replicates were set, and the mean fluorescence intensity was calculated. The data are shown in Table 2 and FIGs. 1 and 2. In FIGs. 1 and 2, the fluorescence intensities are the measured fluorescence intensities corresponding to the tissues of the mice that were photographed using an IVIS small-animal optical *in vivo* imaging system. The fluorescence intensities indicate the levels of luciferase protein expression, i.e., the *in vivo* mRNA delivery efficiency and tissue distribution of the lipid nanoparticle compositions. In FIGs. 1 and 2, * denotes statistical P < 0.05, which indicates that there is a statistically significant difference between the groups.

With the lipid nanoparticle compositions corresponding to comparative compounds 1 and 2 as controls, the mRNA delivery efficiency of the *in vivo* tail vein injection of the lipid nanoparticle compositions corresponding to compounds 1, 2, 3, and 4 into mice was determined.

**Table 2**

| No. | Mean fluorescence intensity (p/s) | |
|---|---|---|
| | 24 h | 48 h |
| Compound 1 | 7.67E+09 | 1.11E+09 |
| Compound 2 | 1.25E+10 | 1.51E+09 |
| Compound 3 | 8.22E+09 | 1.56E+09 |
| Compound 4 | 6.58E+09 | 6.61E+08 |
| Comparative compound 1 | 3.98E+09 | 3.11E+08 |
| Comparative compound 2 | 5.39E+09 | 2.27E+08 |

| | | |
|---|---|---|
| Conclusion: According to the fluorescence intensities shown in Table 2 and FIGs. 1 and 2, the overall fluorescence intensities of the mice injected with the lipid nanoparticle compositions consisting of compounds 1-4 at 24 h and 48 h were significantly higher than those of the mice injected with the lipid nanoparticle compositions corresponding to comparative compounds 1 and 2. This indicates that the lipid nanoparticles corresponding to compounds 1 to 4 and their compositions exhibited better delivery efficiency *in vivo.* The fluorescence intensities and proportions shown in Table 3 and FIG. 4 indicate the levels of protein expression in the organ tissues and the proportions of protein expression in the tissues. The lipid nanoparticle composition consisting of compound 2 exhibited the highest fluorescence intensity in the liver, with the proportion being more than 95%, indicating that lipid nanoparticle compositions corresponding to such compounds have relatively good liver-targeting capability. | | |

**Table 3**

| No. | | Fluorescence intensity (p/s) | Fluorescence intensity proportion (%) |
|---|---|---|---|
| | Heart | 9.52E+05 | 0.03 |
| Compound 2 | Lung | 1.39E+07 | 0.43 |
| | Liver | 3.12E+09 | 95.76 |
| | Spleen | 1.20E+08 | 3.68 |
| | Kidney | 3.48E+06 | 0.10 |

### Test Example 2: Assessment of mRNA Delivery Efficiency of Intramuscular Administration of Lipid Nanoparticle Compositions

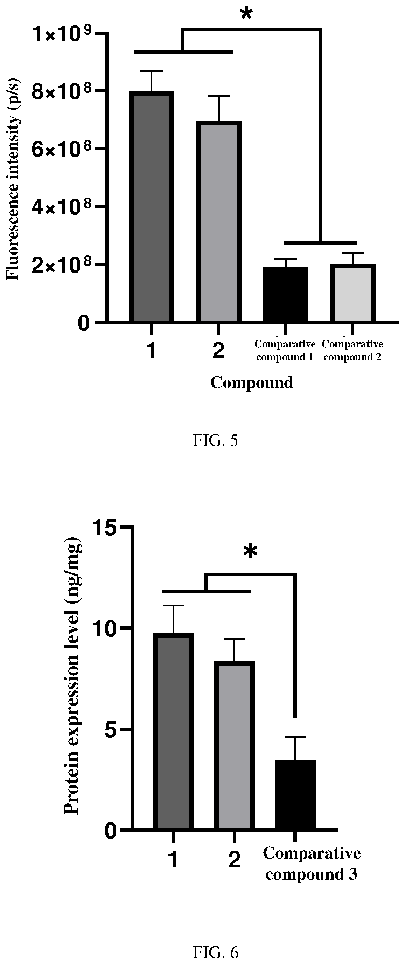

Lipid nanoparticles encapsulating a luciferase-expressing mRNA (GenBank accession No. MN728548.1) were intramuscularly administered to female Balb/C mice aged 6-8 weeks at a dose of 0.1 mg/kg. Twenty-four hours after administration, the luciferase substrate was injected intraperitoneally into each mouse. Fluorescence photos of tissues were taken using an IVIS small-animal optical in vivo imaging system (PerkinElme), and the fluorescence intensity of each tissue was measured. The fluorescence intensity indicates the level of luciferase protein expression. For the lipid nanoparticle composition corresponding to each compound, at least 3 biological replicates were set, and the mean fluorescence intensity was calculated. The data are shown in Table 4 and FIG. 5. In FIG. 5, * denotes statistical P < 0.05, which indicates that there is a statistically significant difference between the groups.

With the lipid nanoparticle compositions corresponding to comparative compounds 1 and 2 as controls, the mRNA delivery efficiency of the *in vivo* intramuscular injection of the lipid nanoparticle compositions corresponding to compounds 1 and 2 into mice was determined.

Conclusion: According to the fluorescence intensities shown in Table 4 and FIG. 5, the fluorescence intensities of the lipid nanoparticle compositions corresponding to compounds 1 and 2 in mice were significantly higher than those of the lipid nanoparticle compositions corresponding to comparative compounds 1 and 2. This indicates that the lipid nanoparticles corresponding to compounds 1 and 2 and their compositions exhibited better delivery efficiency.

**Table 4**

| No. | Mean fluorescence intensity (p/s) |
|---|---|
| Compound 1 | 7.98E+08 |
| Compound 2 | 6.97E+08 |
| Comparative compound 1 | 1.90E+08 |
| Comparative compound 2 | 2.02E+08 |

Lipid nanoparticle compositions in which a hepatocyte growth factor-expressing mRNA (NCBI accession No. NM_000601.6) was encapsulated were injected into the gastrocnemius muscles of female BALB/c mice aged 6-8 weeks at a dose of 0.05 mg/kg. After 6 h, muscle tissue was collected from the injection site, ground, and lysed, and the expression level of hepatocyte growth factor protein (ng/mg), i.e., the amount of hepatocyte growth factor protein corresponding to the total protein amount per unit of muscle tissue, was then determined using an ELISA kit. For the lipid nanoparticle composition corresponding to each compound, at least 3 replicates were set, and the mean protein concentration was calculated.

With the lipid nanoparticle composition corresponding to comparative compound 3 as a control, the mRNA delivery efficiency of the *in vivo* intramuscular injection of the lipid nanoparticle compositions corresponding to compounds 1 and 2 was determined. The related data are shown in FIG. 6 and Table 5.

In FIG. 6, * denotes statistical P < 0.05, which indicates that there is a statistically significant difference between the groups.

**Table 5**

| No. | Mean protein expression level (ng/mg) |
|---|---|
| Compound 1 | 9.73 |
| Compound 2 | 8.39 |
| Comparative compound 3 | 3.45 |

| | |
|---|---|
| Conclusion: According to the protein expression levels shown in Table 5 and FIG. 6, the protein expression levels corresponding to compounds 1 and 2 were significantly higher than that corresponding to comparative compound 3, indicating that the *in vivo* intramuscular injection of the lipid nanoparticle compositions corresponding to compounds 1 and 2 exhibited significantly better effects in delivering the hepatocyte growth factor-expression mRNA than that of the lipid nanoparticle composition corresponding to comparative compound 3. Compound 1 also significantly outperformed compound 2. This indicates that the lipid nanoparticle compositions corresponding to compounds 1 and 2 could more efficiently deliver mRNA and enable protein expression in muscles. | |

### Test Example 3: Assessment of In Vivo Safety of Lipid Nanoparticle Compositions

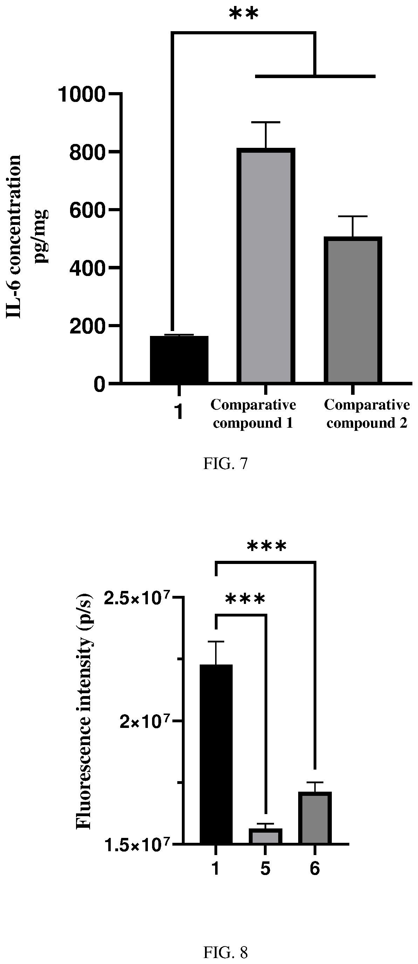

When exogenous substances enter the bodies of mammals, they induce an innate immune response, thereby promoting the production of cytokines. These exogenous substances can cause inflammatory reactions after entry into the body, which easily give rise to adverse reactions, such as fever and edema, in the body. The immunogenicity of lipid nanoparticle compositions in the body was therefore assessed by assessing the concentration of a cytokine, such as interleukin 6 (IL-6), in the blood of mice injected with the lipid nanoparticle compositions. Lower cytokine concentrations indicate that the lipid nanoparticle compositions have lower immunogenicity, i.e., better biological safety. Lipid nanoparticle compositions in which a hepatocyte growth factor protein-expressing mRNA was encapsulated were administered to female BALB/c mice aged 6-8 weeks by intramuscular injection at a dose of 0.05 mg/kg. After 6 h, muscle tissue was collected from the injection site and homogenized. The IL-6 concentration in the muscle tissue, i.e., the amount of IL-6 in a unit of tissue protein (pg/mg), was determined using a mouse IL-6 ELISA kit. For the lipid nanoparticle composition corresponding to each compound, at least 3 biological replicates were set, and the mean IL-6 concentration in serum was calculated. Conclusion: As shown in Table 6 and FIG. 7, the IL6 concentration of the lipid nanoparticle composition corresponding to compound 1 in mouse muscle tissue was significantly lower than those of the lipid nanoparticle compositions corresponding to comparative compounds 1 and 2. This indicates that the lipid nanoparticle composition of compound 1 exhibited lower immunogenicity *in vivo,* which means better biological safety. In FIG. 7, ** indicates a statistically significant difference (P < 0.01).

**Table 6**

| No. | Tissue IL-6 concentration (pg/mg) |
|---|---|
| Compound 1 | 164.2 |
| Comparative compound 1 | 813.1 |
| Comparative compound 2 | 508.1 |

### Test Example 4: Assessment of Freeze-Drying of Lipid Particle Compositions

Lipid nanoparticles were prepared according to the method in Example 1 and exchanged by ultrafiltration into a buffer containing 20 mM Tris, pH 7.5, and 8% sucrose to obtain a freeze-dried formulation solution of the lipid nanoparticle composition. The freeze-dried formulation solution of the lipid nanoparticle composition was added to 2 mL vials (0.6 mL/vial), with the mRNA concentration in each vial being 0.2 mg/mL. The vials were placed in a freeze-dryer and subjected to 3 h of pre-freezing at -45 °C, 30 h of primary drying at -40 °C, and 10 h of secondary drying at 30 °C, with the vacuum degree being 0.05 mmbar. The vials were unloaded from the freeze-dryer, and the final freeze-dried products were obtained.

The final freeze-dried products were taken. After reconstitution with 0.6 mL of water for injection, the particle size and encapsulation efficiency of the lipid nanoparticles were determined according to the aforementioned methods. The particle size of the lipid nanoparticles before freeze-drying was compared with that after freeze-drying. The data are shown below:

**Table 7**

| Test items | Compound 1 | Compound 5 | Compound 6 | Comparative compound 2 |
|---|---|---|---|---|
| Particle size before freeze-drying (nm) | 67.0 | 64.4 | 71.9 | 92.9 |
| Particle size after freeze-drying and reconstitution (nm) | 78.0 | 92.4 | 99.4 | 163.8 |
| Rate of change in particle size after freeze-drying (%) | 16.4 | 43.5 | 38.2 | 76.3 |
| PDI before freeze-drying | 0.04 | 0.10 | 0.16 | 0.14 |
| PDI after freeze-drying | 0.08 | 0.08 | 0.24 | 0.16 |
| Encapsulation efficiency before freeze-drying (%) | 99.5 | 97.9 | 98.5 | 94.0 |
| Encapsulation efficiency after freeze-drying and reconstitution (%) | 92.4 | 80.5 | 84.1 | 82.0 |

The particle size of the lipid nanoparticles corresponding to compound 1 before and after freeze-drying was significantly smaller than that of the lipid nanoparticles corresponding to comparative compound 2 and compounds 5 and 6. Moreover, the increase in particle size after freeze-drying and reconstitution was significantly smaller compared to compounds 5 and 6 and comparative compound 2. In addition, the particle-size distributions (PDI) of the lipid nanoparticles corresponding to compound 1 were all less than 0.1 and were significantly lower than those of the lipid nanoparticles corresponding to compounds 5 and 6 and comparative compound 2. This indicates that the lipid nanoparticles corresponding to compound 1 exhibited more uniform particle size distributions, and could still maintain uniform particle size distributions after freeze-drying. Upon reconstitution after freeze-drying, the encapsulation efficiency of compound 1 was still higher than 90%. In contrast, upon reconstitution after freeze-drying, the encapsulation efficiency of the lipid nanoparticles corresponding to comparative compound 2 and compounds 5 and 6 significantly decreased and was lower than 85%, which is the general encapsulation efficiency quality control standard.

This indicates that the lipid nanoparticles corresponding to compound 1 and their composition could withstand freeze-thaw cycles and temperature changes during freeze-drying, making them suitable for the freeze-drying process.

### Test Example 5: Assessment of mRNA Delivery Efficiency of Lipid Nanoparticle Compositions on Cells

The protein expression levels of the lipid nanoparticle compositions corresponding to compounds 1, 5, and 6 after mRNA delivery in cells were determined.

HEK 293 cells were seeded in 96-well plates and cultured overnight. When the cell density reached 80% or higher, solutions of lipid nanoparticle compositions in which the luciferase mRNA (GenBank accession No. MN728548.1) was encapsulated were added to the culture medium in the wells of the cell plates, with the mRNA dose being 100 ng/well. After 24 h, the fluorescence intensity of the expressed luciferase protein was measured using a luciferase reporter gene assay kit (Promega) and a microplate reader. The fluorescence intensity values, i.e., the fluorescence measurements obtained using the microplate reader, represent expression levels of luciferase protein. The higher the fluorescence intensity, the higher the protein expression level. For the lipid nanoparticles corresponding to each compound, at least 3 replicates were set, and the mean fluorescence intensity was calculated.

**Table 8**

| No. | Mean fluorescence intensity |
|---|---|
| Compound 1 | 2.23E+07 |
| Compound 5 | 1.56E+07 |
| Compound 6 | 1.71E+07 |

| | |
|---|---|
| Conclusion: According to the fluorescence intensities shown in the table and FIG. 8, the expression level of the lipid nanoparticle composition consisting of compound 1 was significantly higher than those of the lipid nanoparticle compositions consisting of compounds 5 and 6, indicating that compound 1 exhibited better delivery efficiency. | |

In FIG. 8, *** indicates a statistically significant difference (P < 0.001).

HuH-7 cells (human liver cancer cells) were seeded in 96-well plates and cultured overnight. When the cell density reached 80% or higher, solutions of lipid nanoparticle compositions in which the luciferase mRNA (GenBank accession No. MN728548.1) was encapsulated were added to the culture medium in the wells of the cell plates, with the mRNA dose being 100 ng/well. After 24 h, the fluorescence intensity of the expressed luciferase protein was measured using a luciferase reporter gene assay kit (Promega) and a microplate reader. The fluorescence intensity values, i.e., the fluorescence measurements obtained using the microplate reader, represent expression levels of luciferase protein. The higher the fluorescence intensity, the higher the protein expression level. For the lipid nanoparticle composition corresponding to each compound, at least 3 replicates were set, and the mean fluorescence intensity was calculated.

**Table 9**

| No. | Mean fluorescence intensity |
|---|---|
| Compound 1 | 6.48E+07 |
| Compound 5 | 4.54E+07 |
| Compound 6 | 5.19E+07 |
| Comparative compound 1 | 4.18E+07 |

| | |
|---|---|
| Conclusion: According to the fluorescence intensities shown in Table 9 and FIG. 9, the expression level of the lipid nanoparticle composition consisting of compound 1 was significantly higher than those of the lipid nanoparticle compositions consisting of compounds 5 and 6 and comparative compound 1, indicating that compound 1 exhibited better delivery efficiency. The expression level of the lipid nanoparticle composition consisting of compound 5 was not significantly different from that of the lipid nanoparticle composition consisting of comparative compound 1. | |

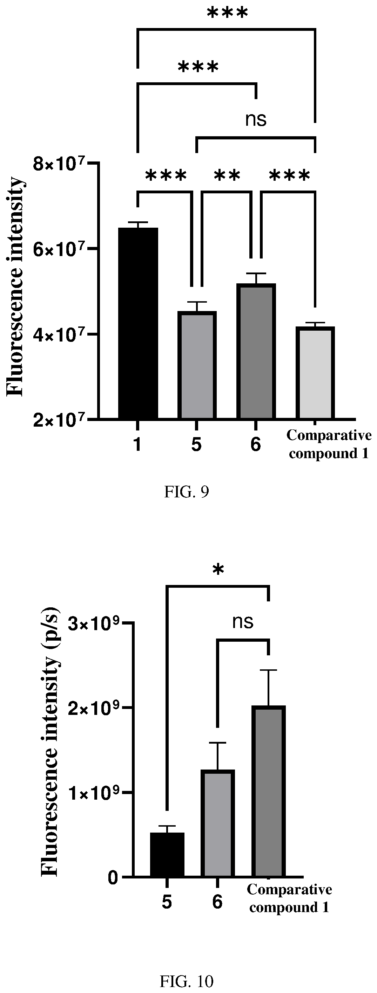

In FIG. 9, ** denotes statistical P < 0.01, and *** denotes statistical P < 0.001, and they indicate that there are statistically significant differences between the groups.

### Test Example 6: Assessment of mRNA Delivery Efficiency of In Vivo Tail Vein Injection of Lipid Nanoparticle Compositions

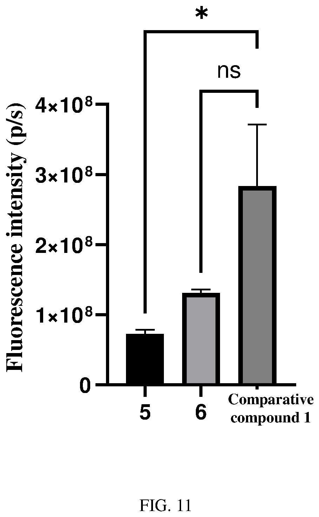

Lipid nanoparticle compositions encapsulating a luciferase-expressing mRNA (GenBank accession No. MN728548.1) were administered to female Balb/c mice aged 6-8 weeks by tail vein injection at a dose of 0.5 mg/kg. Twenty-four hours and forty-eight hours after administration, the luciferase substrate was injected intraperitoneally into each mouse. Fluorescence photos of tissues were taken using an IVIS small-animal optical in vivo imaging system (PerkinElme), and the fluorescence intensity was measured. The fluorescence intensity indicates the level of luciferase protein expression, i.e., the *in vivo* mRNA delivery efficiency of the lipid nanoparticles. For the lipid nanoparticle composition corresponding to each compound, at least 3 biological replicates were set, and the mean fluorescence intensity was calculated. The data are shown in Table 10 and FIGs. 10 and 11. In FIGs. 10 and 11, the fluorescence intensities are the measured fluorescence intensities corresponding to the tissues of the mice that were photographed using an IVIS small-animal optical *in vivo* imaging system. The fluorescence intensities indicate the levels of luciferase protein expression, i.e., the *in vivo* mRNA delivery efficiency and tissue distribution of the lipid nanoparticle compositions.

In FIGs. 10 and 11, * denotes statistical P < 0.05, which indicates that there is a statistically significant difference between the groups. ns denotes statistical P ≥ 0.05, which indicates that there is no statistically significant difference between the groups.

With the lipid nanoparticle composition corresponding to comparative compound 1 as a control, the mRNA delivery efficiency of the *in vivo* tail vein injection of the lipid nanoparticle compositions corresponding to compounds 5 and 6 into mice was determined.

**Table 10**

| No. | Mean fluorescence intensity (p/s) | |
|---|---|---|
| | 24 h | 48 h |
| Compound 5 | 5.25E+08 | 7.26E+07 |
| Compound 6 | 1.27E+09 | 1.31E+08 |
| Comparative compound 1 | 2.02E+09 | 2.84E+08 |

| | | |
|---|---|---|
| Conclusion: According to the fluorescence intensities shown in Table 10 and FIGs. 10 and 11, the overall fluorescence intensities of the mice injected with the lipid nanoparticle composition consisting of compound 5 at 24 h and 48 h were significantly lower than those of the mice injected with the lipid nanoparticle composition corresponding to comparative compound 1. | | |

## Claims

1. A compound represented by formula I or a salt thereof, 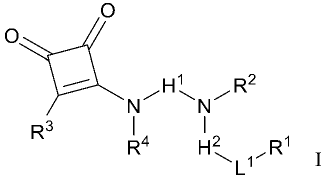
wherein H¹ is selected from the group consisting of C₁₋₁₂ alkylene, C₂₋₁₂ alkenylene, C₃₋₈ cycloalkylene, and C₃₋₈ cycloalkenylene;
H² is selected from the group consisting of C₁₋₁₂ alkylene and C₁₋₁₂ heteroalkylene;
provided that when H² is C₁₋₁₂ alkylene, H¹ is selected from the group consisting of C₃₋₈ cycloalkylene and C₃₋₈ cycloalkenylene;
R¹ is selected from the group consisting of C₁₋₂₄ alkyl and C₂₋₂₄ alkenyl;
R² is selected from the group consisting of C₁₋₂₄ alkyl, C₁₋₂₄ alkenyl, and -R^{b}-L²-R^{c};
each L¹ or L² is independently selected from the group consisting of -C(O)O-, -OC(O)-, -C(O)-, -OC(O)O-, -O-, -S(O)q-, -S-S-, -C(O)S-, -SC(O)-, -NR^{a}C(O)-, -C(O)NR^{a}-, - NR^{a}C(O)NR^{a}-, -NR^{a}C(O)O-, -OC(O)NR^{a}-, -P(O)(OR^{a})-, and a bond, wherein R^{a} is selected from the group consisting of hydrogen and C₁₋₆ alkyl, and q is selected from the group consisting of 0, 1, and 2;
R^{b} is selected from the group consisting of C₁₋₁₂ alkylene and C₂₋₁₂ alkenylene;
R^{c} is selected from the group consisting of C₁₋₂₄ alkyl and C₂₋₂₄ alkenyl;
R³ is selected from the group consisting of alkylamino and cycloalkylamino;
R⁴ is selected from the group consisting of hydrogen and C₁₋₆ alkyl.

2. The compound or the salt thereof according to claim 1, wherein H² is C₁₋₁₂ heteroalkylene, preferably C₂₋₉ heteroalkylene.

3. The compound or the salt thereof according to claim 1 or 2, wherein R³ is alkylamino, preferably methylamino or dimethylamino.

4. The compound or the salt thereof according to any one of claims 1-3, wherein R¹ is C₂₋₂₄ alkyl, preferably C₄₋₁₈ alkyl; or R¹ is C₂₋₂₄ alkenyl, preferably C₄₋₁₈ alkenyl.

5. The compound or the salt thereof according to any one of claims 1-4, wherein R² is - R^{b}-L²-R^{c}, wherein R^{b}, L², and R^{c} are as defined in claim 1.

6. The compound or the salt thereof according to claim 1 or 5, wherein the compound represented by formula I is a compound represented by formula IIa: 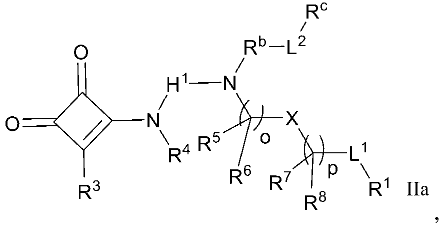 wherein X is -N(R^{d})- or -O-; o is selected from the group consisting of integers between 1 and 11, p is selected from the group consisting of integers between 1 and 5, and o + p is not greater than 12; R^{d} is selected from the group consisting of hydrogen and C₁₋₆ alkyl; R⁵, R⁶, R⁷, and R⁸ are each independently selected from the group consisting of hydrogen, halogen, hydroxy, and C₁₋₆ alkyl; L¹, L², H¹, R¹, R³, R⁴, R^{b}, and R^{c} are as defined in claim 1.

7. The compound or the salt thereof according to claim 6, wherein p is selected from the group consisting of 1 and 2.

8. The compound or the salt thereof according to claim 6, wherein o is selected from the group consisting of integers between 4 and 7, such as 5 or 6.

9. The compound or the salt thereof according to any one of claims 1-8, wherein H¹ is C₁₋₁₂ alkylene, preferably C₃₋₈ alkylene.

10. The compound or the salt thereof according to claim 1, wherein H¹ is selected from the group consisting of C₃₋₈ cycloalkylene; preferably, the compound represented by formula I is a compound represented by formula IIb: 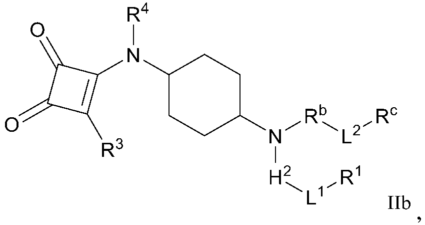 wherein L¹, L², H², R¹, R³, R⁴, R^{b}, and R^{c} are as defined in claim 1.

11. The compound or the salt thereof according to any one of claims 1-10, wherein each L¹ or L² is independently selected from the group consisting of -C(O)O-, -OC(O)-, and a bond.

12. The compound or the salt thereof according to claim 1, 6, or 10, wherein R^{b} is C₁₋₁₂ alkylene.

13. The compound or the salt thereof according to claim 1, 6, or 10, wherein R^{c} is C₁₋₂₄ alkyl.

14. A compound represented by formula A or a salt thereof: 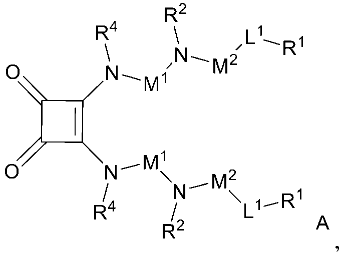 wherein each M¹ is independently selected from the group consisting of C₁₋₁₂ alkylene, C₂₋₁₂ alkenylene, C₃₋₈ cycloalkylene, and C₃₋₈ cycloalkenylene;
each M² is independently selected from the group consisting of C₁₋₁₂ alkylene and C₁₋₁₂ heteroalkylene;
each R¹ is independently selected from the group consisting of C₁₋₂₄ alkyl and C₂₋₂₄ alkenyl;
each R² is independently selected from the group consisting of C₁₋₂₄ alkyl, C₁₋₂₄ alkenyl, and -R^{b}-L²-R^{c};
each L¹ or L² is independently selected from the group consisting of -C(O)O-, -OC(O)-, -C(O)-, -OC(O)O-, -O-, -S(O)q-, -S-S-, -C(O)S-, -SC(O)-, -NR^{a}C(O)-, -C(O)NR^{a}-, - NR^{a}C(O)NR^{a}-, -NR^{a}C(O)O-, -OC(O)NR^{a}-, -P(O)(OR^{a}), and a bond, wherein R^{a} is selected from the group consisting of hydrogen and C₁₋₆ alkyl and C₂₋₆ alkenyl, and q is selected from the group consisting of 0, 1, and 2;
each R^{b} is independently selected from the group consisting of C₁₋₁₂ alkylene and C₂₋₁₂ alkenylene;
each R^{c} is independently selected from the group consisting of C₁₋₂₄ alkyl and C₂₋₂₄ alkenyl;
each R⁴ is independently selected from the group consisting of hydrogen and C₁₋₆ alkyl.

15. The compound or the salt thereof according to any one of claims 1-14, wherein R¹ or R^{c} is selected from the group consisting of: 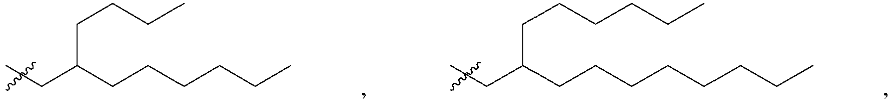 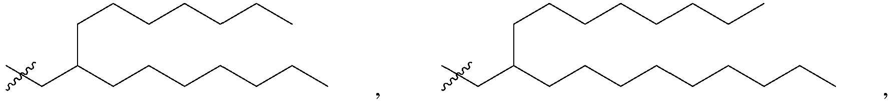 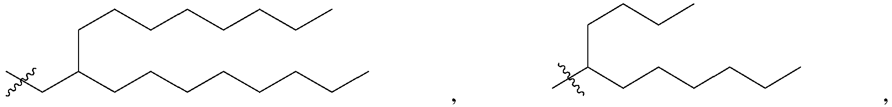 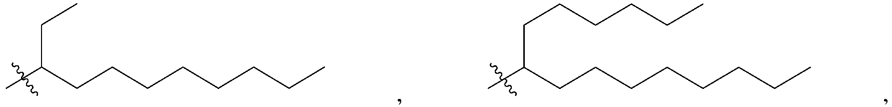 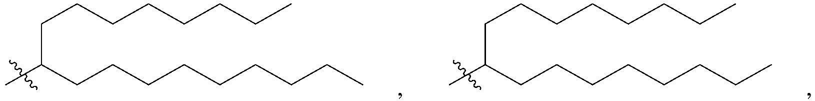 and

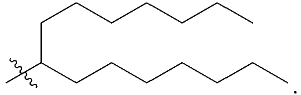

16. The compound or the salt thereof according to claim 1 or claim 14, wherein the compound represented by formula I is selected from the group consisting of: 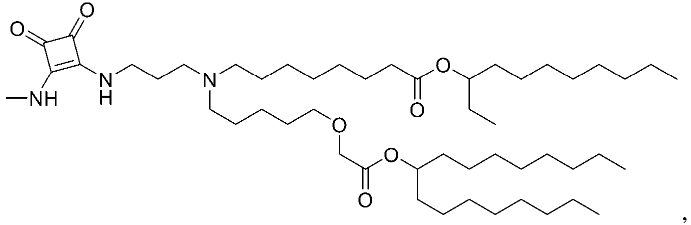 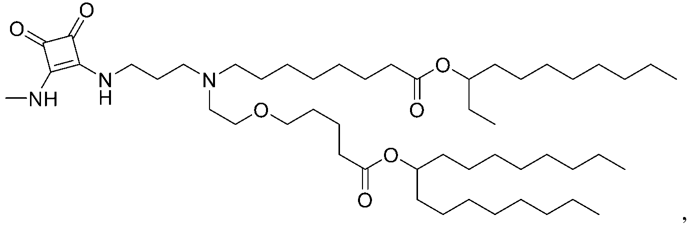 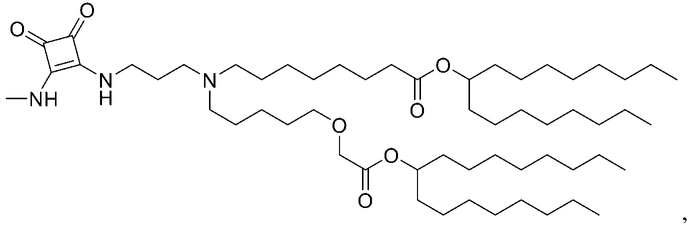 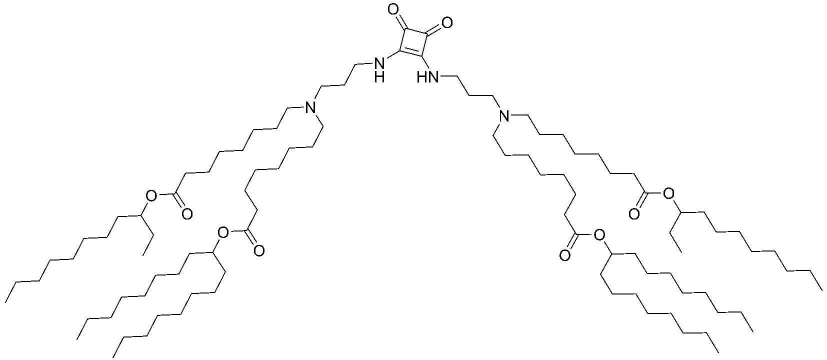 and

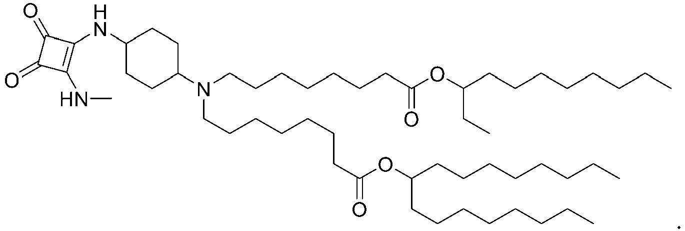

17. An isotopically substituted form of the compound according to any one of claims 1-16, wherein preferably, the isotopically substituted form is a deuterium-substituted form.

18. A lipid particle, comprising the compound or the salt thereof according to any one of claims 1-16 or an isotopically substituted form thereof, wherein the lipid particle further comprises an active agent, and the active agent is preferably a polynucleotide or a nucleic acid, e.g., a DNA, an siRNA, or an mRNA.

19. A pharmaceutical composition, comprising the lipid particle according to claim 18 and a pharmaceutically acceptable excipient.

20. Use of the compound or the salt thereof according to any one of claims 1-16, or the isotopically substituted form according to claim 17, or the lipid particle according to claim 18, or the pharmaceutical composition according to claim 19 in the manufacture of a medicament for preventing and/or treating cancer, infection, autoimmune diseases, neurodegenerative diseases, and inflammation.
